# FASCICULE DE BREVET EUROPEEN

(11) **EP 1 086 080 B1**
(45) Date de publication et mention de la délivrance du brevet: **25.08.2004**
(21) Numéro de dépôt: 99923723.3
(22) Date de dépôt: 11.06.1999
(51) Int. Cl.: C07C 391/02, C07D 213/80, A61K 33/04, A61K 7/40, C07D 335/06, C07D 409/12

(54) **COMPOSES DIARYLSELENURES ET LEUR UTILISATION EN MEDECINE HUMAINE OU VETERINAIRE AINSI QU'EN COSMETOLOGIE**
DIARYLSELENID-VERBINDUNGEN UND IHRE VERWENDUNG IN MENSCHLICHE ODER TIERISCHE MEDIZIN WIE IN KOSMETOLOGIE
DIARYLSELENIDE COMPOUNDS AND THEIR USE IN HUMAN OR VETERINARY MEDICINE AND IN COSMETICS

(30) Priorité: 12.06.1998 FR 9807439
(43) Date de publication de la demande: 28.03.2001
(73) Titulaire: Galderma Research & Development, S.N.C., 06560 Valbonne (FR)
(72) Inventeur: BERNARDON, Jean-Michel, F-06650 Le Rouret (FR); DIAZ, Philippe, 06200 Nice (FR)
(74) Mandataire: Renard, Emmanuelle
(86) Numéro de dépôt international: PCT/FR1999/001389
(87) Numéro de publication internationale: WO 1999/065872

(56) Documents cités:
- EP-A- 0 661 258
- EP-A- 0 679 630
- EP-B- 0 170 105
- WO-A-92/20643
- WO-A-93/21146
- WO-A-94/12880
- WO-A-97/16422
- WO-A-98/22423
- M.F. BOEHM ET AL.: JOURNAL OF MEDICINAL CHEMISTRY, vol. 38, 1995, pages 3146-3155, XP002115271 WASHINGTON US
- KAGECHIKA H ET AL: "RETINOBENZOIC ACIDS. STRUCTURE-ACTIVITY RELATIONSHIPS OF AROMATIC AMIDES WITH RETINOIDAL ACTIVITY" JOURNAL OF MEDICINAL CHEMISTRY, vol. 31, no. 11, 1 novembre 1988 (1988-11-01), pages 2182-2192, XP000608417 ISSN: 0022-2623
- YU K -L ET AL: "STRUCTURAL MODIFICATIONS OF 6-NAPHTHALENE-2-CARBOXYLATE RETINOIDS" BIOORGANIC & MEDICINAL CHEMISTRY LETTERS, vol. 6, no. 23, 1 janvier 1996 (1996-01-01), pages 2865-2870, XP002057399 ISSN: 0960-894X
- PEMRICK S M ET AL: "THE RETINOID RECEPTORS" LEUKEMIA, vol. 8, no. SUPPL. 03, 1 janvier 1994 (1994-01-01), pages S01-S10, XP000566434
- CHEMICAL ABSTRACTS, vol. 126, no. 2, 13 janvier 1997 (1997-01-13) Columbus, Ohio, US; abstract no. 18962, XP002096532 & NAKANISHI WARO ET AL.: CHEM. LETT., vol. 11, 1996, pages 947-948,

## Description

L'invention concerne, à titre de produits industriels nouveaux et utiles, des composés diarylsélénures. Elle concerne également l'utilisation de ces nouveaux composés dans des compositions pharmaceutiques destinées à un usage en médecine humaine ou vétérinaire, ou bien encore dans des compositions cosmétiques.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des coméopathies.

Les demandes de brevet EP 0 679 630 et WO 98/22423 et l'article de Boehm *et al.* (Journal of Medicinal Chemistry, vol. 38, 1995, p.3146-3155) décrivent tous trois une nouvelle classe de lignads RXR bicycliques aromatiques.
La demande de brevet EP 0 679 630 décrit ces composés comme présentant une activité sur l'expression de certains marqueurs de la différentiation des kératinocytes humains in vitro et présentant une bonne activité comédolytique dans le test des souris Rhino. Dans la demande de brevet WO 98/22423, il est indiqué que ces composés ont une activité sur la différentiation et la prolifération cellulaire, enfin, l'article de Boehm *et al.* souligne leur intérêt pour le traitement de cancers et de désordres dermatologiques.

Cette classe de composés diffère des composés objets de la présente invention par le fait que les deux cycles aromatiques sont séparés par un atome d'oxygène, de soufre ou d'azote ou bien par une liaison carbonée complexe alors que dans les composés selon l'invention, les deux cycles sont séparés par un atome de sélénium.

On peut également utiliser les composés selon l'invention dans des compositions cosmétiques pour l'hygiène corporelle et capillaire.

La présente invention concerne des composés qui peuvent être représentés par la formule générale (I) suivante : 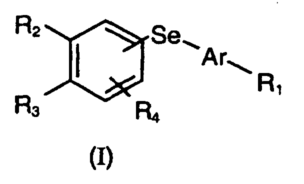 dans laquelle :
- R₁ représente :
   (i) le radical -CH₃
   (ii) le radical -CH₂-O-R₅
   (iii) le radical -COR₆
      R₅ et R₆ ayant les significations données ci-après,
- Ar représente un radical choisi parmi les radicaux de formules (a)-(e) suivantes : 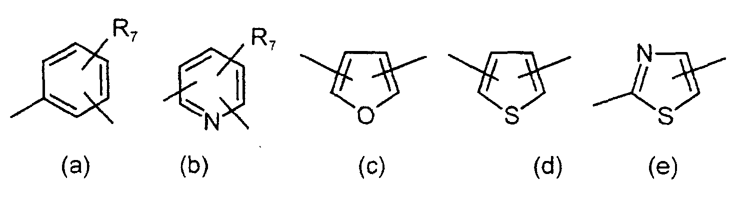 R₇ ayant la signification donnée ci-après,
- R₂ et R₃ identiques ou différents indépendamment représentent un radical choisi parmi:
   (i) un atome d'hydrogène,
   (ii) un radical choisi parmi les radicaux tertiobutyle, 1-méthylcyclohexyl, ou 1-Adamantyl,
   (iii) un radical -OR₈, R₈ ayant la signification donnée ci-après,
   (iv) un radical polyéther,
   étant entendu qu'au moins un des radicaux R₂ ou R₃ représentent un radical (ii),
- R₂ et R₃ pris ensemble peuvent former avec le cycle aromatique adjacent un cycle saturé à 5 ou 6 chaînons éventuellement substitué par des groupes méthyles et / ou éventuellement interrompu par un atome d'oxygène ou de soufre,
- R₄ représente un atome d'hydrogène, un atome d'halogène, un radical alkyle inférieur, un radical OR₉, un radical polyéther, ou un radical COR₁₀,
   R₉ et R₁₀ ayant les significations données ci-après,
- R₅ représente un atome d'hydrogène, un radical alkyle inférieur, ou un radical COR₁₁
   R₁₁ ayant la signification donnée ci-après,
- R₆ représente un radical choisi parmi :
   (i) un atome d'hydrogène,
   (ii) un radical alkyle inférieur,
   (iii) un radical OR₁₂
      R₁₂ ayant la signification donnée ci-après,
   (iv) un radical de formule 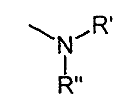 R' et R" ayant les significations données ci-après,
- R₇ représente un atome d'hydrogène, un atome d'halogène, un radical alkyle inférieur, un radical nitro, un radical OR₁₃, un radical polyéther ou un radical de formule suivante : 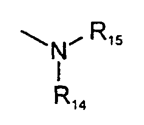 R₁₃, R₁₄, R₁₅ ayant les significations données ci-après,
- R₈ représente un atome d'hydrogène, un radical alkyle inférieur, un radical aryle éventuellement substitué, un radical aralkyle éventuellement substitué, un radical monohydroxyalkyle ou polyhydroxyalkyle, ou un radical acyle inférieur,
- R₉ représente un atome d'hydrogène, un radical alkyle inférieur, un radical aryle éventuellement substitué, un radical aralkyle éventuellement substitué, un radical monohydroxyalkyle ou polyhydroxyalkyle, un radical acyle inférieur, un radical -(CH₂)n-COOR₁₆, ou un radical -(CH₂)n-X,
   n, R₁₆ et X ayant les significations données ci-après,
- R₁₀ et R₁₁, identiques ou différents, représentent un radical alkyle inférieur,
- R₁₂ représente un atome d'hydrogène, un radical alkyle inférieur, un radical aryle ou aralkyle éventuellement substitué, un radical monohydroxyalkyle ou un radical polyhydroxyalkyle,
- R' et R", identiques ou différents, représentent un atome d'hydrogène, un radical alkyle inférieur, un radical aryle éventuellement substitué, ou un reste d'aminoacide,
   ou encore R' et R" pris ensemble peuvent former avec l'atome d'azote un hétérocycle,
- R₁₃ représente un atome d'hydrogène, ou un radical alkyle inférieur,
- R₁₄ et R₁₅, identiques ou différents, représentent un atome d'hydrogène, ou un radical alkyle inférieur,
- R₁₆ représente un atome d'hydrogène, ou un radical alkyle inférieur,
   n représente un entier compris entre 1 et 12 inclus,
- X représente un atome d'halogène,

L'invention vise également les sels des composés de formule (I) lorsque R, représente une fonction acide carboxylique et les isomères géométriques et optiques desdits composés de formule (I).
Lorsque les composés selon l'invention se présentent sous forme de sels, il s'agit de préférence de sels d'un métal alcalin ou alcalino-terreux, ou encore de zinc ou d'une amine organique.
Selon la présente invention on entend par radical alkyle inférieur un radical ayant de 1 à 6 atomes de carbone et de préférence les radicaux méthyle, éthyle, isopropyle, butyle et tertiobutyle.

Par radical monohydroxyalkyle, on entend un radical ayant de 1 à 6 atomes de carbone, notamment un radical 2-hydroxyéthyle, 2-hydroxypropyle ou 3-hydroxypropyle, le radical monohydroxyalkyle pouvant être protégé sous forme d'acétyle ou de tert-butyldiméthylsilyle
Par radical polyhydroxyalkyle, on entend un radical contenant de 2 à 6 atomes de carbone et de 2 à 5 groupes hydroxyles tels que notamment les radicaux 2,3-dihydroxypropyle, 2,3,4-trihydroxybutyle, 2,3,4,5-tétrahydroxypentyle ou le reste du pentaérythritol, les groupes hydroxyles pouvant être protégés sous forme d'acétyles ou de tert-butyldiméthylsilyles.
Par radical aryle éventuellement substitué, on entend un radical phényle éventuellement substitué par au moins un atome d'halogène, un hydroxyle optionnellement protégé sous la forme d'une fonction éther ou acétate, une fonction nitro, ou une fonction amino éventuellement substituée par un groupement alkyl ou acétyl.
Par radical aralkyle éventuellement substitué, on entend un radical benzyle ou un radical phénéthyle éventuellement substitué par au moins un atome d'halogène, un radical hydroxyle optionnellement protégé sous la forme d'une fonction éther ou acétate, une fonction nitro, ou une fonction amino éventuellement substituée par un groupement alkyl ou acétyl.
Par radical acyle inférieur, on entend un radical ayant de 1 à 4 atomes de carbone, et de préférence un radical acétyle ou propionyle.
Par reste d'aminoacide on entend un reste dérivant par exemple de l'un des 20 aminoacides de configuration L ou D constitutifs de protéines de mammifères.
Par hétérocycle, on entend de préférence un radical pipéridino, morpholino, pyrrolidino ou pipérazino, éventuellement substitué en position 4 par un radical alkyle en C₁-C₆ ou par un mono ou polyhydroxyalkyle tels que définis ci-dessus.
Par radical polyéther, on entend un radical ayant de 1 à 6 atomes de carbone et de 1 à 3 atomes d'oxygène ou de soufre, tels que les radicaux méthoxyméthyl éther, méthoxyéthoxyméthyl éther ou méthylthiométhyl éther.

Par atome d'halogène, on entend de préférence un atome de fluor, de chlore ou de brome.

Selon la présente invention, les composés de formule (I) plus particulièrement préférés sont ceux pour lesquels l'une au moins et de préférence toutes les conditions ci-dessous sont respectées :
- R₁ représente un radical COR₆
- Ar représente un radical de formule (a) ou (b)
- R₂ ou R₃ représentent un radical adamantyle ou R₂ et R₃ pris ensemble forment avec le cycle aromatique adjacent un cycle saturé à 5 ou 6 chaînons éventuellement substitué par des groupes méthyle et / ou éventuellement interrompu par un atome d'oxygène ou de soufre.

Parmi les composés de formule (I) ci-dessus rentrant dans le cadre de la présente invention, on peut citer notamment les suivants :
4-(3,5,5,8,8-Pentamethyl-5,6,7,8-tetrahydro-naphthalen-2-ylselanyl)-benzoate d'ethyle,
acide 4-(3,5,5,8,8-Pentamethyl-5,6,7,8-tetrahydro-naphthalen-2-ylselanyl)-benzoique,
6-(3,5,5,8,8-Pentamethyl-5,6,7,8-tetrahydro-naphthalen-2-ylselanyl)-nicotinate d'ethyle,
acide 6-(3,5,5,8,8-pentaméthyl-5,6,7,8-tetrahydro-naphtalen-2-ylselanyl)-nicotinique,
6-(5,5,8,8-Tetramethyl-3-propoxy-5,6,7,8-tetrahydro-naphthalen-2-ylselanyl)-nicotinate d'ethyle,
acide 6-(5,5,8,8-Tetramethyl-3-propoxy-5,6,7,8-tetrahydro-naphthalen-2-ylselanyl-nicotinique,
acide 3-(4-tert-Butyl-phenylselanyl)-benzoique,
acide 6-(4-tert-Butyl-phenylselanyl)-nicotinique,
acide 4-(4-tert-Butyl-phenylselanyl)-benzoique,
acide 4-(4,4-Dimethyl-thiochroman-8-ylselanyl)-benzoique,
acide 3-(4,4-Dimethyl-thiochroman-8-ylselanyl)-benzoique,
acide 6-(4,4-Dimethyl-thiochroman-8-ylselanyl)-nicotinique.
acide 4-(5,5,8,8-Tetramethyl-5,6,7,8-tetrahydro-naphthalen-2-ylselanyl)-benzoique,
acide 3-(5,5,8,8-Tetramethyl-5,6,7,8-tetrahydro-naphthalen-2-ylselanyl)-benzoique,
acide 6-(5,5,8,8-Tetramethyl-5,6,7,8-tetrahydro-naphthalen-2-ylsetanyl)-nicotinique,
acide 4-[5-Adamantan-1-yl-4-(2-methoxy-ethoxymethoxy)-2-methylphenylselanyl] -benzoique,
acide 3-[5-Adamantan-1-yl-4-(2-methoxy-ethoxymethoxy)-2-methylphenylselanyl] -benzoique,
acide 6-(4-Méthoxyéthoxyméthoxy-5,5,8,8-tétraméthyl-5,6,7,8-tétrahydronaphthalen-2-ylselanyl)-nicotinique,
acide 3-(4-Méthoxyéthoxyméthoxy-5,5,8,8-tétraméthyl-5,6,7,8-tétrahydronaphthalen-2-ylselanyl)-benzoïque,
acide 4-(4-Méthoxyéthoxyméthoxy-5,5,8,8-tétraméthyl-5,6,7,8-tétrahydronaphthalen-2-ylselanyl)-3-méthoxy-benzoïque,
acide 3-(4-Méthoxyéthoxyméthoxy-5,5,8,8-tétraméthyl-5,6,7,8-tétrahydronaphthalen-2-ylselanyl)-4-méthoxy-benzoïque,
acide 6-(4-Méthoxyméthoxy-5,5,8,8-tétraméthyl-5,6,7,8-tétrahydro-naphthalen-2-ylselanyl)-nicotinique,
acide 6-(3-Méthoxyéthoxyméthoxy-5,5,8,8-tétraméthyl-5,6,7,8-tétrahydronaphthalen-2-ylselanyl)-nicotinique,
acide 2-(3-Méthoxyéthoxyméthoxy-5,5,8,8-tétraméthyl-5,6,7,8-tétrahydronaphthalen-2-ylselanyl)-nicotinique,
acide 4-(3-Méthoxyéthoxyméthoxy-5,5,8,8-tétraméthyl-5,6,7,8-tétrahydronaphthalen-2-ylselanyl)-benzoïque,
acide 3-(3-Méthoxyéthoxyméthoxy-5,5,8,8-tétraméthyl-5,6,7,8-tétrahydronaphthalen-2-ylselanyl)-benzoïque,
acide 6-(3,5-Di-tert-butyl-2-méthoxyméthoxy-phénylselanyl)-nicotinique,
acide 2-(3,5-Di-tert-butyl-2-méthoxyméthoxy-phénylselany4)-nicotinique,
acide 4-(3,5-Di-tert-butyl-2-méthoxyméthoxy-phénylselanyl)-benzoïque,
acide 3-(3,5-Di-tert-butyl-2-méthoxyméthoxy-phénylselanyl)-benzoïque,
acide 6-[4-adamantan-1-yl-3-benzyloxy-phenylsélenalyl]-nicotinique,
acide 6-(3,5-Di-tert-butyl-2-benzyloxy-phénylselanyl)-nicotinique,
acide 3-méthoxy-4-(4-benzyloxy-5,6,7,8-tétrahydro-5,5,8,8-tétraméthylnaphtalen-2-ylsélanyl)-benzoïque,
acide 4-(4-benzyloxy-5,6,7,8-tétrahydro-5,5,8,8-tétraméthylnaphtalen-2-ylsélanyl)-benzoïque,
acide 6-(4-benzyloxy-5,6,7,8-tétrahydro-5,5,8,8-tétraméthylnaphtalen-2-ylséfanyl)-nicotinique,
acide 3-méthoxy-4-(3-benzyloxy-5,6,7,8-tétrahydro-5,5,8,8-tétraméthylnaphtalen-2-ylsélanyl)-benzoïque,
acide 6-(3-benzyloxy-5,6,7,8-tétrahydro-5,5,8,8-tétraméthylnaphtalen-2-ylsélanyl)-nicotinique,
acide 4-(3-hexyloxy-5,6,7,8-tétrahydro-5,5,8,8-tétraméthylnaphtalen-2-ylsélanyl)3-méthoxy-benzoïque,
acide 6-(3-hexyloxy-5,6,7,8-tétrahydro-5,5,8,8-tétraméthylnaphtalen-2-ylsélanyl)-nicotinique,
acide 4-(5-adamantan-1-yl-4-benzyloxy-2-méthyl-phenylselenalyl)-benzoïque,
acide 6-[3-(5-Hydroxy-pentyloxy)-5,5,8,8-tetramethyl-5,6,7,8-tetrahydronaphthalen-2-ylselanyl]- nicotinique,
4-(5,5,8,8-Tetramethyl-5,6,7,8-tetrahydro-naphthalen-2-ylselanyl)-benzoate d'éthyle,
4-(3-Méthoxyéthoxyméthoxy-5,5,8,8-tétraméthyl-5,6,7,8-tétrahydro-naphthalen-2-ylselanyl)-benzoate d'éthyle,
4-(3-Hydroxy-5,5,8,8-tetramethyl-5,6,7,8-tetrahydro-naphthalen-2-ylselanyl)-benzoate d'éthyle,
acide 4-(3-Hydroxy-5,5,8,8-tetramethyl-5,6,7,8-tetrahydro-naphthalen-2-ylselanyl) -benzoïque,
6-(3-Méthoxyéthoxyméthoxy-5,5,8,8-tétraméthyl-5,6,7,8-tétrahydro-naphthalen-2-ylselanyl)-nicotinate d'éthyle,
6-(3-Hydroxy-5,5,8,8-tetramethyl-5,6,7,8-tetrahydro-naphthalen-2-ylselanyl)-nicotinate d'éthyle,
acide 6-(3-Hydroxy-5,5,8,8-tetramethyl-5,6,7,8-tetrahydro-naphthalen-2-ylselanyl) -nicotinique,
6-[3-(3-Ethoxycarbonyl-propoxy)-5,5,8,8-tetramethyl-5,6,7,8-tetrahydronaphthalen-2- ylselanyl]-nicotinate d'éthyle,
acide 6-[3-(3-Carboxy-propoxy)-5,5,8,8-tetramethyl-5,6,7,8-tetrahydronaphthalen -2-ylselanyl]- nicotinique,
4-[3-(3-Ethoxycarbonyl-propoxy)-5,5,8,8-tetramethyl-5,6,7,8-tetrahydronaphthalen-2- ylselanyl]-benzoate d'éthyle,
acide 4-[3-(3-Carboxy-propoxy)-5,5,8,8-tetramethyl-5,6,7,8-tetrahydronaphthalen -2-ylselanyl]- benzoique,
4-[3-(7-Methoxycarbonyl-heptyloxy)-5,5,8,8-tetramethyl-5,6,7,8-tetrahydronaphthalen-2- ylselanyl]-benzoate d'éthyle,
acide 4-[3-(7-Carboxy-heptyloxy)-5,5,8,8-tetramethyl-5,6,7,8-tetrahydronaphthalen-2-ylselanyl]- benzoique,
6-[3-(7-Methoxycarbonyl-heptyloxy)-5,5,8,8-tetramethyl-5,6,7,8-tetrahydronaphthalen-2- ylselanyl]-nicotinate d'éthyle,
acide 6-[3-(7-Carboxy-heptyloxy)-5,5,8,8-tetramethyl-5,6,7,8-tetrahydronaphthalen-2-ylselanyl]- nicotinique,
6-[3-(2-Acetoxy-ethoxy)-5,5,8,8-tetramethyl-5,6,7,8-tetrahydro-naphthalen-2-ylselanyl]- nicotinate d'éthyle,
acide 6-[3-(2-Hydroxy-ethoxy)-5,5,8,8-tetramethyl-5,6,7,8-tetrahydronaphthalen-2-ylselanyl]- nicotinique,
4-[3-(2-Acetoxy-ethoxy)-5, 5,8,8-tetramethyl-5,6,7,8-tetrahydro-naphthalen-2-ylselanyl]-benzoate d'éthyle,
acide 4-[3-(2-Hydroxy-ethoxy)-5,5,8,8-tetramethyl-5,6,7,8-tetrahydronaphthalen-2-ylselanyl]-benzoique,
acide 6-(3-Adamantan-1-yl-4-methoxy-phenylselanyl)-nicotinique, [6-(3,5,5,8,8-Pentamethyl-5,6,7,8-tetrahydro-naphthalen-2-ylselanyl)-pyridin-3-yl]-methanol,
N-Ethyl-6-(3,5,5,8,8-pentamethyl-5,6,7,8-tetrahydro-naphthalen-2-ylselanyl)-nicotinamide,
Morpholin-4-yl-[6-(3,5,5,8,8-pentamethyl-5, 6, 7, 8-tetrahydro-naphthalen-2-ylselanyl)-pyridin-3-yl]-methanone,
N-(4-Hydroxy-phenyl)-6-(3,5,5,8,8-pentamethyl-5,6,7,8-tetrahydro-naphthalen-2-ylselanyl)-nicotinamide,
6-(3,5,5,8,8-Pentamethyl-5,6,7,8-tetrahydro-naphthalen-2-ylselanyl)-pyridine-3-carbaldehyde,

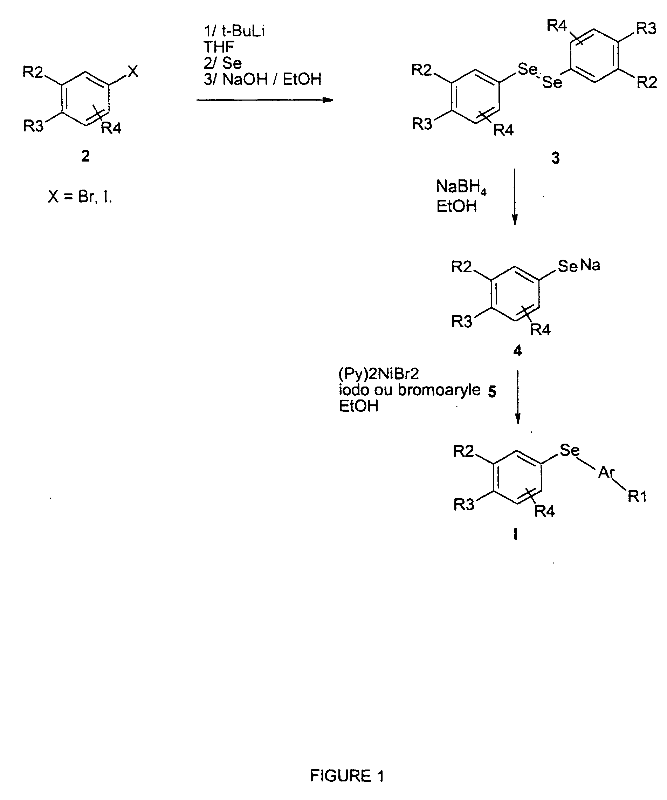

La présente invention a également pour objet les procédés de préparations des composés de formule (1), en particulier selon le schéma réactionnel donné à la figure 1.
Les dérivés de formule (I) peuvent être obtenus (FIG. 1) par une suite de réactions comprenant, l'action d'une base lithiée telle que le tBuLi sur le produit **(2)** dans un solvant comme le THF, suivie de l'addition de sélénium et de la formation du dimère par oxydation en milieu basique (EtOH, NaOH). Le produit **(3)** obtenu est soumis à l'action du borohydrure de sodium dans un solvant comme l'éthanol puis couplé avec un iodoaryle en présence d'un catalyseur au nickel.
Lorsque R₁ représente le radical COOH, les composés sont préparés en protégeant R₁ par un groupe protecteur de type alkyle. La saponification de la fonction ester en présence d'une base, telle l'hydroxyde de sodium ou de lithium dans un solvant alcoolique ou dans le THF conduit aux acides correspondants.
Lorsque R₁ représente un radical alcool, les composés peuvent être obtenus à partir de l'acide par réduction en présence d'hydrure comme l'hydrure de bore. L'alcool peut être ethérifié selon les méthode classiques.
Lorsque R₁ représente un radical aldéhyde, les composés peuvent être obtenus par oxydation des alcools correspondants par action d'oxyde de manganèse ou de pyridinium dichromate.
Lorsque R₁ représente un radical amide, les composés peuvent être obtenus par transformation de l'acide en chlorure d'acide puis par réaction avec une amine appropriée.

Ces composés se lient aux récepteurs RXRs, les uns possédant une activité agoniste, les autres une activité antagoniste.
Les propriétés de fixation et de transactivation comme agoniste aux récepteurs RXRs peuvent être déterminées par des méthodes connues dans l'art, comme par exemple : LEVIN et al, Nature 1992, **355**, 359-61 ; ALLENBY et al, Proc. Natl. Acad. Sci., 1993, **90**, 30-4.
L'activité agoniste RXRs peut être aussi déterminée par le test tel que décrit dans la demande de brevet français n° 95-07301 déposée le 19 juin 1995 par la demanderesse. Ce test comprend les étapes suivantes : (i) on applique topiquement sur une partie de la peau d'un mammifère une quantité suffisante d'un composé qui est un ligand actif d'au moins un récepteur de la superfamille des récepteurs nucléaires stéroïdiens/thyroïdiens autre qu'un ligand spécifique des récepteurs RXRs et pouvant s'hétérodimériser avec les RXRs tel qu'une molécule agoniste des RARs, (ii) on administre par voie systémique ou topique sur cette même partie de la peau du mammifère avant, pendant ou après l'étape (i) une molécule susceptible de présenter une activité agoniste des RXRs, (iii) on évalue la réponse sur la partie de la peau ainsi traitée du mammifère. Ainsi la réponse à une application topique sur l'oreille d'un mammifère d'une molécule agoniste des RARs qui correspond à une augmentation de l'épaisseur de cette oreille peut être augmentée par l'administration par voie systémique ou topique d'une molécule agoniste des récepteurs RXRs.

L'activité antagoniste RXRα peut être évaluée dans le test de transactivation par détermination de la dose (IC₅₀) qui inhibe de 50% l'activité transactivatrice d'un agoniste sélectif RXRα: l'acide 6-[1-(3,5,5,8,8-pentaméthyl-5,6,7,8-tétrahydronaphtalen-2-yl)cyclopropyl]nicotinique (CD 3127) selon le protocole suivant :
Les cellules Hela sont co-transfectées avec un vecteur d'expression codant pour RXRα (p565-RXRα) et un plasmide rapporteur contenant l'élément de réponse 1/2 CRBP II cloné en amont du promoteur hétérologue de la thymidine kinase et du gène rapporteur de la chloramphénicolm-acétyl-transfèrase (CAT). Dix-huit heures après co-transfection les cellules sont traitées avec une concentration fixe du CD 3127 et des concentrations croissantes de la molécule à évaluer. Après vingt-quatre heures de traitement le dosage de l'activité CAT est effectué par ELISA. La concentration fixe de CD3127 utilisée est 10⁻⁸M et correspond à son EC₅₀.
La présente invention a ainsi pour objet à titre de médicament les composés de formule (I) telle que définie ci-dessus.
Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique, ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation, pour prévenir ou pour réparer les vergetures, ou encore pour favoriser la cicatrisation,
10) pour lutter contre les troubles de la fonction sébacée tels que l'hyperséborrhée de l'acné ou la séborrhée simple,
11) dans le traitement ou la prévention des états cancéreux ou précancéreux, plus particulièrement les leucémies promyélocytaires,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose, l'hypertension, le diabète non-insulino dépendant ainsi que l'obésité,
17) dans le traitement de désordres cutanés dus à une exposition aux rayonnements U.V..

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy
ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutase, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique, les dérivés de l'acide salicylique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.
La présente invention a également pour objet des compositions médicamenteuses contenant au moins un composé de formule (I) telle que définie ci-dessus, l'un de ses isomères optiques ou géométriques ou un de ses sels.
La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (1), l'un de ses isomères optiques ou géométriques ou un de ses sels.
L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.
Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.
Par voie oculaire, ce sont principalement des collyres.
Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.
Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.
Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.
La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.
La concentration en composé de formule (I) dans les compositions cosmétiques selon l'invention est avantageusement comprise entre 0,001% et 3% en poids par rapport à l'ensemble de la composition.
Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3- méthyl 1,2,4-benzothiadiazine 1,1-dioxyde) et le Phénytoïn (5,5-diphényl-imidazolidine 2,4-dione); des agents anti-inflammatoires non stéroïdiens; des caroténoïdes et, notamment, le β-caroténe; des agents anti-psoriatiques tels que l'anthraline et ses dérivés; et enfin les acides eicosa-5,8,11,14-tétraynoïque et eicosa-5,8,11-trynoïque, leurs esters et amides.
Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés.

### A. EXEMPLES DE COMPOSES

### EXEMPLE 1 :

### 4-(3,5,5,8,8-Pentamethyl-5,6,7,8-tetrahydro-naphthalen-2-ylselanyl)-benzoate d'ethyle

(a) 5,6,7,8-tetrahydro-3,5,5,8,8-pentamethylnaphtalene-2-disélénide
   Du tert-butyllithium 1,7 M dans le pentane (37,4 mmol, 22 ml) est additionnée à une solution de 2-bromo-5,6,7,8-tetrahydro-3,5,5,8,8-pentaméthylnaphtalene (4,4 g, 15,8 mmol) dans le THF (100 ml) à -78°C en 10 min. Le mélange est agité à 0°C 30 min. Le sélénium(1,33 g, 16,8 mmol) est additionné en 2 fois. Le mélange est agité à 0°C 15 min, puis à température ambiante 30 min. Une solution d'HCl 1N (40 ml) est additionnée, puis le mélange réactionnel est traité par de l'éther éthylique. La phase organique est lavée 2 fois à l'eau, séchée sur sulfate de magnésium anhydre et concentrée à l'évaporateur rotatif sous vide à 40°C. 10 ml d'éthanol et 50 mg de soude sont additionnés à l'huile obtenue. Le mélange est agité vigoureusement quelques minutes à l'air (qsp tout précipite), puis est concentré à l'évaporateur rotatif sous vide à 40°C. Le solide obtenu est filtré sur silice (élution heptane) puis cristallisé dans un mélange éthanol / éther. Solide jaune. Masse : 3,26 g. Rendement : 74%. Tf: 126°C.
   RMN1H (CDCl₃) : 1,14 (6H, s), 1,23 (6H, s), 1,61 (4H, s), 2,35 (3H, s), 7,05 (1H Ar, s), 7,55 (1H Ar, s).
(b) 4-(3,5,5,8,8-Pentamethyl-5,6,7,8-tetrahydro-naphthalen-2-ylselanyl)-benzoate d'ethyle
   Une solution de 5,6,7,8-tetrahydro-3,5,5,8,8-pentamethylnaphtafene-2-disélénide (500 g, 0,89 mmol) et de borohydrure de sodium (68 mg, 1,8 mmol) dans 5 ml d'éthanol est agitée 1 heure à température ambiante. On ajoute, ensuite, du iodobenzoate d'éthyle (440 mg, 1,6 mmol) et du Bis(bipyridine)nickel 2 bromide (10 mg, 0,016 mmol) (Organometallics 1985, 4, 657-661 ). La solution est chauffée 5 minutes à reflux. A température ambiante, elle est diluée par de l'éther éthylique. La phase organique est lavée à l'eau, séchée sur sulfate de magnésium anhydre, puis concentrée. Le résidu est purifié par fast plug (éluant: heptane puis éther éthylique).
   Solide blanc. Masse: 495 mg. Rendement: 72%. Tf: 104°C.
   RMN 1H (CDCl₃): 1,22 (6H, s), 1,29 (6H, s), 1,33-1,39 (3H, t), 1,67 (4H, s), 2,32 (3H, s), 4,29-4,38 (2H, q), 7,21-7,26 (3H, c), 7,51 (1H, s), 7,84-7,87 (2H, d).

### EXEMPLE 2 :

### Acide 4-(3,5,5,8,8-Pentamethyl-5,6,7,8-tetrahydro-naphthalen-2-ylselanyl)-benzoïque.

De la soude (450 mg, 11,25 mmol) est additionnée à la solution de 4-(3,5,5,8,8-Pentamethyl-5,6,1,8-tetrahydro-naphthalen-2-ylselanyl)-benzoate d'ethyle (450 mg, 1,04 mmol) dans un mélange de 10 ml de THF, 1 ml de méthanol et 1 ml d'eau. Le milieu réactionnel est chauffé 12 h à reflux. Il est ensuite versé sur un mélange éther éthylique / eau, acidifié à pH 1 par une solution d'acide chlorhydrique concentré et extrait à l'éther éthylique. Après décantation, la phase organique est lavée 2 fois à l'eau, séchée sur sulfate de magnésium anhydre et concentrée à l'évaporateur rotatif sous vide à 40°C.
Poudre blanche. Masse: 371 mg. Rendement: 88%. Tf: 249°C.
RMN 1H (CDCl₃): 1,21 (6H, s), 1,29 (6H, s), 1,67 (4H, s), 2,32 (3H, s), 7,21-7,24 (2H, d, J= 6,9 Hz), 7,38 (1H, s), 7,48 (1H, s), 785-7,88 (2H, d, J= 8,35 Hz).

### EXEMPLE 3 :

### 6-(3,5,5,8,8-Pentamethyl-5,6,7,8-tetrahydro-naphthalen-2-ylselanyl)-nicotinate d'ethyle

De manière analogue à l'exemple 1(b), par réaction de 750 mg (1,33 mmol) de disélénide dans 15 ml d'éthanol avec 102 mg (2,7 mmol) de borohydrure de sodium, 665 mg (2,4 mmol) de 6-iodonicotinate d'éthyle et 15 mg (0,024 mmol) de Bis (bipyridine) nickel 2 bromide, on obtient 779 mg (75%) du dérivé attendu sous forme d'un solide blanc. Tf: 117°C.
RMN 1H (CDCl₃): 1,25 (6H, s), 1,31 (6H, s), 1,34-1,40 (3H, t), 1,69 (4H, s), 2,37 (3H, s), 4,32-4,40 (2H, q), 6,83-6,87 (1H, d, J=8,3 Hz), 7,28 (1H, s), 7,65 (1H, s), 7,91-7,96 (1H, dd, J= 6,10 Hz, J'= 2,21Hz), 8,99-9,00 (1H, d, J= 2,14 Hz).

### EXEMPLE 4 :

### Acide 6-(3,5,5,8,8-pentaméthyl-5,6,7,8-tetrahydro-naphtalen-2-ylselanyl)-nicotinique

De manière analogue à l'exemple 2, par réaction de 750 mg (1,74 mmol) de 6-(3,5,5,8,8-Pentamethyl-5,6,7,8-tetrahydro-naphthalen-2-ylselanyl)-nicotinate d'ethyle avec 700 mg (17,5 mmol) de soude dans un mélange THF / méthanol / eau, on obtient 625 mg (89%) de coton blanc. Tf :258°C.
RMN 1H (DMSO) : 1,05 (6H, s), 1,11 (6H, s), 1,48 (4H, s), 2,14 (3H, s), 6,79-6,83 (1H, d, J= 8,3 Hz), 7,24 (1H, s), 7,45 (1H, s), 7,83-7,88 (1H , dd, J= 6,03 Hz, J'= 2,3 Hz), 8,69-8,70 (1H, d, J= 2,2 Hz), 13,12 (1H, s).

### EXEMPLE 5 :

### 6-(5,5,8,8-Tetramethyl-3-propoxy-5,6,7,8-tetrahydro-naphthalen-2-ylselanyl)-nicotinate d'ethyle

(a) 1,1,4,4-tetraméthyl-7-propoxy-1,2,3,4-tetrahydronaphtalen-6-disélénide
   De manière analogue à l'exemple 1 (a), par réaction de 6 g (18,5 mmol) de 6-bromo-1,1,4,4-tetraméthyl-7-propoxy-1,2,3,4-tetrahydronaphtalene avec du tert-Butyllithium 1,7 M dans le pentane et du Sélénium dans 20 ml de THF, on obtient 3,2 g du dérivé sélénié attendu sous forme d'un solide jaune. Tf: 92-98°C.
   RMN 1H (CDCl₃): 1,05-1,10 (6H, m), 1,25 (9H, m), 1,55-1,66 (4H, m), 1,86 (2H, sext), 3,98 (2H, t), 6,67 (1H, s), 7,42 (1H, s).
(b) 6-(5,5,8,8-Tetramethyl-3-propoxy-5,6,7,8-tetrahydro-naphthalen-2-ylselanyl)-nicotinate d'ethyle
   De manière analogue à l'exemple 1(b), par réaction de 850 mg (1,31 mmol) de disélénide dans 85 ml d'éthanol avec 120 mg (2,62 mmol) de borohydrure de sodium, 581 mg (2,1 mmol) de 6-iodonicotinate d'éthyle et 20 mg (0,032 mmol) de Bis (bipyridine) nickel 2 bromide, on obtient 610 mg (61%) du composé attendu sous forme de cristaux blancs. Tf: 110-12°C.
   RMN1H (CDCl₃): 0,81-0,87 (3H, t), 1,24 (6H, s), 1,31 (6H, s), 1,35-1,41 (3H, t), 1,57-1,65 (2H, m), 1,69 (4H, s), 3,87-3,92 (2H, t), 4,32-4,41 (2H, q), 6,66 (1H, s), 7,00-7,03 (1H, d, J= 8,3 Hz), 7,59 (1H, s), 7,91-7,95 (1H, dd, J= 6,2 Hz, J'= 2,1Hz), 8,98-8,99 (1H, d, J= 1,7 Hz).

### EXEMPLE 6 :

### Acide 6-(5,5,8,8-Tétramethyl-3-propoxy-5,6,7,8-tétrahydro-naphthalen-2-ylselanyl)-nicotinique

De manière analogue à l'exemple 2, par réaction de 485 mg (1,02 mmol) de 6-(5,5,8,8-tétraméthyl-3-propoxy-5,6,7,8-tétrahydro-naphthalen-2-ylselanyl)-nicotinate d'ethyle avec 385 mg (9,6 mmol) de soude dans l'éthanol (20ml), on obtient 444 mg (97%) de solide blanc. Tf :220°C.

### EXEMPLE 7:

### Acide 3-(4-tert-butyl-phenylselenalyl)-benzoïque

Un mélange de 4-*tert*-butyl-phenyl-diselenide (0,3 mmol) de 480 mg de borohydride polymer supported sur résine amberlyst IRA 400 à 2,5 mmol/g (Aldrich), de dibromure de Bis(bipyridine)nickel II (5mg) (Organometallics 1985, 4, 657-661) et de 3-iodobenzoate d'éthyle (0,4 mmol) est chauffé 12 h. à 67°C. Le mélange est filtré et la solution est concentrée. Le solide obtenu est purifié sur cartouche SPE chargée de gel de silice. Les fractions contenant le produit attendu sont réunies et concentrées sous vide. L'ester est saponifié dans un mélange de 2,5 ml de THF, de 2,5 ml d'alcool éthylique et de 0,5 ml d'une solution aqueuse de soude à 33%. Le milieu réactionnel est acidifié par une solution d'HCl, extrait à l'éther éthylique, séché sur sulfate de magnésium et concentré pour donner le produit attendu
RMN ¹H/CDCl₃: 1.32(s, 9H); 7.32 à 7.38 (m, 3H); 7.46 (d, 2H); 7.61 (d,1H); 7.95 (d,1H); 8.19 (d,1H)

### EXEMPLE 8 :

### Acide 6-(4-tert-butyl-phenylselenalyl)-nicotinique

Le produit est obtenu de manière analogue à l'exemple 7, à partir de 4-*tert*-butyl-phenyl-diselenide et de 6-iodo-nicotinate d'éthyle.
RMN ¹H/CDCl₃:1.36(s, 9H); 7.02 (d, 1H); 7.45 (d, 2H); 7.65 (d,2H); 7.96 (d,1H); 9.05 (d,1H).

### EXEMPLE 9 :

### Acide 4-(4-tert-butyl-phenylselenalyl)-benzoïque

Le produit est obtenu de manière analogue à l'exemple 7, à partir de 4-*tert*-butyl-phenyl-diselenide et de 4-iodo-benzoate d'éthyle.
RMN ¹H/CDCl₃:1.34 (s, 9H); 7.35 (d, 2H); 7.39 (d, 2H), 7.54 (d, 2H); 7.92 (d,2H).

### EXEMPLE 10 :

### Acide 4-(4,4-diméthyl-thiochroman-8-ylselenalyl)-benzoïque

(a) 2-bromo-1-(3-méthylbut-2-ènylthio)benzène.
   Dans un tricol, on introduit 19,30 g (102,0 mmoles) de 2-bromothiophénol, 160 ml de DMF et 15,50 g (112,0 mmoles) de carbonate de potassium. On ajoute goutte à goutte 13 ml (112,0 mmoles) de 1-bromo-3-méthyl-2-butène et agite à la température ambiante pendant deux heures. On verse le milieu réactionnel dans l'eau, extrait avec de l'acétate d'éthyle, décante la phase organique, lave à l'eau, sèche sur sulfate de magnésium, évapore. On recueille 26,00 g (99%) du composé attendu, sous la forme d'une huile orangée.
   ¹H NMR (CDCl₃) d 1,65 (s, 3H), 1,73 (s, 3H), 3,56 (d, 2H, J = 7,7 Hz), 5,32 (td, 1H, J = 7,7 /1,4 Hz), 6,96 à 7,06 (m, 1H), 7,22 à 7,26 (m, 2H), 7,52 (d, 1H, J = 7,7 Hz).
(b) 4,4-diméthyl-8-bromothiochromane
   Dans un tricol, on introduit 26,00 g (102,0 mmoles) de 2-bromo-1-(3-méthylbut-2-ènylthio)benzène, 180 ml de toluène et 23,20 g (122,0 mmoles) d'acide para-toluène sulfonique. On chauffe à reflux pendant quatre heures et évapore le milieu réactionnel à sec. On reprend par une solution aqueuse d'hydrogénocarbonate de sodium, extrait avec de l'acétate d'éthyle, décante la phase organique, sèche sur sulfate de magnésium, évapore. Le résidu obtenu est purifié par chromatographie sur colonne de silice, élué avec de l'heptane. On recueille 20,00 g (76%) du composé attendu, sous la forme d'une huile orangée.
   ¹H NMR (CDCl₃) d 1,33 (s, 6H), 1,94 (t, 2H, J = 6,0 Hz), 3,04 (t, 2H, J = 6,1 Hz), 6,89 (t, 1H, J = 7,9 Hz), 7,34 (d, 2H, J = 7,9 Hz).
(c) 4,4-Dimethyl-thiochroman-8-disélénide.
   Un cristal d'iode, du magnésium (208 mg, 8,56 mmol) et quelques gouttes d'une solution de 4,4-diméthyl-8-bromothiochromane (2g, 7,78 mmol) dans l'éther éthylique (15ml) sont chauffés jusqu'à l'amorçage de l'organomagnésien. Le reste de la solution est alors additionnée goutte à goutte. Le milieu réactionnel est chauffé 2h, puis le sélénium (615 mg, 7,78 mmol) est additionné à température ambiante. L'agitation est poursuivie 30 mn puis, une solution d'HCl 1N est additionnée. Le mélange réactionnel est traité par de l'éther éthylique. La phase organique est lavée 2 fois à l'eau, séchée sur sulfate de magnésium anhydre et concentrée à l'évaporateur rotatif sous vide à 40°C. De l'éthanol et de l'hydroxyde de sodium sont additionnés à l'huile obtenue. Le mélange est agité vigoureusement quelques minutes, puis est concentré à l'évaporateur rotatif sous vide à 40°C.
   Le produit est purifié sur colonne de silice (dichlorométhane 20 - heptane 80).
   Solide blanc. Masse 300 mg. Rendement : 15%.
   RMN 1H (CDCl₃) : 1,33 (6H, s), 1,96 (2H, m), 3,09 (2H, m), 6,93 (1H Ar, t, J=7,8 Hz), 7,26 (1H Ar, dd, J=7,8 Hz, J=1,3 Hz), 7,47 (1H Ar, dd, J=7,8 Hz, J=1,3 Hz).
(d) Acide 4-(4,4-dimethyl-thiochroman-8-ylselenalyl)-benzoïque
   Le produit est obtenu de manière analogue à l'exemple 7, à partir de 4,4-dimethyl-thiochroman-8-diselenide et de 4-ioda-benzoate d'éthyle.
   RMN ¹H/CDCl₃:1.36 (s, 6H); 1.95 (m, 2H), 2.99 (m, 2H), 6.99 (t, 1H), 7.31 à 7.46 (m, 4H); 7.91 (d, 2H).

### EXEMPLE 11 :

### Acide 3-(4,4-diméthyl-thiochroman-8-ylselenalyl)-benzoïque

Le produit est obtenu de manière analogue à l'exemple 7, à partir de 4,4-dimethyl-thiochroman-8-diselenide et de 3-iodo-benzoate d'éthyle.
RMN ¹H/CDCl₃:1.35 (s, 6H); 1.95 (m, 2H), 3.02 (m, 2H), 6.94 (t, 1H), 7.18 (dd, 1H); 7.33 à 7.39 (m, 2H), 7.61 (dd, 1H), 8.08 (dd, 1H), 8.16 (d, 1H).

### EXEMPLE 12 :

### Acide 6-(4,4-diméthyl-thiochroman-8-ylselenalyl)-nicotinique

Le produit est obtenu de manière analogue à l'exemple 7, à partir de 4,4-dimethyl-thiochroman-8-diselenide et de 6-iodo-nicotinate d'éthyle.
RMN ¹H/CDCl₃:1.37 (s, 6H); 1.95 (m, 2H), 2.97 (m, 2H), 6.90 (d, 1H), 7.04 (t, 1H); 7.48 à 7.57 (m, 2H), 7.96 (dd, 1H), 9.03 (d, 1H).

### EXEMPLE 13 :

### Acide 4-(5,5,8,8-tétraméthyl-5,6.7,8-tétrahydronaphthalen-2-ylselenalyl)-benzoïque

(a) 5,6,7,8-tetrahydro-5,5,8,8-tétramethylnaphtalene-2-disélénide.
   Une solution de tert-butyllithium 1,7 M dans le pentane (37,4 mmol, 22 ml) est additionnée à une solution de 2-bromo-5,6,7,8-tetrahydro-5,5,8,8-tetraméthylnaphtalene (4,22 g, 15,8 mmol) dans le THF (100 ml) à -78°C en 10 min. Le mélange est agité à 0°C 30 min. Le sélénium(1,33 g, 16,8 mmol) est additionné en 2 fois. Le mélange est agité à 0°C 15 min, puis à température ambiante 30 min. Une solution d'HCl 1N (40 ml) est additionnée, puis le mélange réactionnel est traité par de l'éther éthylique. La phase organique est lavée 2 fois à l'eau, séchée sur sulfate de magnésium anhydre et concentrée à l'évaporateur rotatif sous vide à 40°C. 10 ml d'éthanol et 50 mg de soude sont additionnés à l'huile obtenue. Le mélange est agité vigoureusement quelques minutes à l'air (qsp tout précipite), puis est concentré à l'évaporateur rotatif sous vide à 40°C. Le solide obtenu est filtré sur silice (élution heptane) puis cristallisé dans un mélange éthanol / éther.
   Solide orange. Masse : 2,9 g. Rendement : 69%.
   RMN ¹H (CDCl₃): 1,21 (6H, s), 1,25 (6H, s), 1,65 (4H, s), 7,20 (1H Ar, d, J=8,25 Hz), 7,38 (1H Ar, dd ,J=1,9 Hz, J=8,25 Hz), 7,51 (1H Ar, d, J=1,9 Hz).
(b) Acide 4-(5,5,8,8-tetramethyl-5,6,7,8-tetrahydronaphthalen-2-ylselenalyl)-benzoïque.
   Le produit est obtenu de manière analogue à l'exemple 7, à partir de 5,6,7,8-tetrahydro-5,5,8,8-tetramethylnaphtalene-2-disélénide et de 4-iodo-benzoate d'éthyle.
   RMN ¹H/CDCl₃:1.26 (s, 6H); 1.30 (s, 6H), 1.70 (s, 4H), 7.27 à 7.37 (m, 4H), 7.54 (d, 1H), 7.91 (d, 2H).

### EXEMPLE 14 :

### Acide 3-(5,5,8,8-tétraméthyl-5,6,7,8-tétrahydronaphthalen-2-ylsélenalyl)-benzoïque

Le produit est obtenu de manière analogue à l'exemple 7, à partir de 5,6,7,8-tétrahydro-5,5,8,8-tétramethylnaphtalene-2-disélénide et de 4-iodo-benzoate d'éthyle.
RMN ¹H/CDCl₃:1.25 (s, 6H); 1.27 (s, 6H), 1.68 (s, 4H), 7.24 à 7.26 (m, 2H), 7.34 (t, 1H), 7.48 (s, 1H), 7.60 (dd, 1H), 7.94 (dd, 1H), 8.19 (d, 1H).

### EXEMPLE 15 :

### Acide 6-(5,5,8,8-tétraméthyl-5,6,7,8-tétrahydronaphthalen-2-ylselenalyl)-nicotinique

Le produit est obtenu de manière analogue à l'exemple 7, à partir de 5,6,7,8-tetrahydro-5,5,8,8-tétraméthylnaphtalene-2-disélénide et de 6-iodo-nicotinate d'éthyle.
RMN ¹H/CDCl₃:1.29 (s, 6H); 1.32 (s, 6H), 1.72 (s, 4H), 7.03 (s, 1H), 7.36 (d, 1H), 7.45 (dd, 1H), 7.65 (d, 1H), 7.99 (dd, 1H), 9.07 (d, 1H).

### EXEMPLE 16 :

### Acide 4-[5-adamantan-1-yl-4-(2-méthoxyéthoxymethoxy)-2-méthyl-phenyl selenalyl)-benzoïque

a) 5-Adamantan-1-yl-4-(2-méthoxy-éthoxyméthoxy)-2-méthyl-phenyl disélénide. Une petite portion d'une solution de 2-(adamantan-1-yl)-4-bromo-5-methyl-1-méthoxyéthoxyméthoxyphényl (17 g, 41,5 mmol) dans le THF (160 ml) est coulée sur un mélange de magnésium (1,51 g) et un cristal d'iode, en chauffant légèrement. Quand le milieu réactionnel se décolore le reste de la solution est additionnée de manière à maintenir un léger reflux. Après la fin de l'addition, la solution est chauffée à reflux 1 h. Après retour à température ambiante, 3,6 g de sélénium sont additionnés. Le milieu réactionnel est agité 3 h. à température ambiante, puis une solution d'acide chlorhydrique 1N (105 ml) et de l'éther éthylique sont additionnés au milieu réactionnel. La phase organique est lavée à l'eau, séchée sur sulfate de magnésium et concentrée à l'évaporateur rotatif. Puis, on additionne de l'hydroxyde de sodium (131 mg) et de l'éthanol (27 ml). La suspension est agitée à l'air et à température ambiante 12 h. Le produit est purifié par filtration sur silice élué avec du dichlorométhane. On obtient 12 g (71 %) d'un solide jaune. Tf=101°C.
   RMN 1H/CDCl3: 1.73 (s,6H); 2.00 (s,9H); 2.30 (s,3H); 3.40 (s,3H); 3.59 (m,2H); 3.83 (m,2H); 5.29 (s,2H); 6.95 (s,1H); 7.48 (s, 1H).
b) Acide 4-[5-adamantan-1-yl-4-(2-méthoxyéthoxyméthoxy)-2-méthyl-phenyl sélenalyl)-benzoïque
   Le produit est obtenu de manière analogue à l'exemple 7, à partir de 5-Adamantan-1-yl-4-(2-méthoxy-éthoxyméthoxy)-2-méthyl-phenyl disélénide et de 4-iodo-benzoate d'éthyle.
   RMN ¹H/CDCl₃:1.75 (s, 6H); 2.07 (s, 9H), 2.34 (s, 3H), 3.42 (s, 3H), 3.62 (m, 2H), 3.89 (m, 2H), 5.35 (s, 2H), 7.14 (s, 1H), 7.19 (d, 2H), 7.50 (s, 1H), 7.87 (d, 2H).

### EXEMPLE 17 :

### Acide 3-[5-adamantan-1-yl-4-(2-méthoxyéthoxyméthoxy)-2-méthyl-phenyl sélenalyl]-benzoïque

Le produit est obtenu de manière analogue à l'exemple 7, à partir de 5-Adamantan-1-yl-4-(2-méthoxy-éthoxyméthoxy)-2-méthyl-phenyl disélénide et du 3-iodo-benzoate d'éthyle.
RMN ¹H/CDCl₃:1.75 (s, 6H); 2.06 (s, 9H), 2.34 (s. 3H), 3.41 (s, 3H), 3.62 (m, 2H), 3.87 (m, 2H), 5.34 (s, 2H), 7.10 (s, 1H), 7.28 (t, 1H), 7.38 (dd, 1H), 7.47 (s, 1H), 7.87 (dd, 1H), 8.02 (d, 1H).

### EXEMPLE 18 :

### Acide 6-(4-Méthoxyéthoxyméthoxy-5,5,8,8-tétraméthyl-5,6,7,8-tétrahydronaphthalen-2-ylselanyl)-nicotinique

a) 4-méthoxyéthoxymethoxy-5,6,7,8-tetrahydro-5,5,8,8-tetramethylnaphtalene-2-disélénide
   De manière analogue à l'exemple 1(a), à partir du 2-bromo-5,5,8,8-tetramethyl-4-methoxyéthoxymethoxy-5,6,7,8-tetrahydro-naphthalen, on obtient le composé attendu sous forme d'une huile orange.
b) Acide 6-(4-Méthoxyéthoxyméthoxy-5,5,8,8-tétraméthyl-5,6,7,8-tétrahydronaphthalen-2-ylselanyl)-nicotinique
   Le produit est obtenu de manière analogue à l'exemple 7, à partir de 4-méthoxyéthoxyméthoxy-5,6,7,8-tetrahydro-5,5,8,8-tétramethylnaphtalene-2-disélénide et du 6-iodo-nicotinate d'éthyle.
   RMN ¹H/CDCl₃:1.27 (s, 6H); 1.42 (s, 6H), 1.67 (m, 4H), 3.36 (s, 3H), 3.56 (m, 2H), 3.82 (m, 2H), 5.29 (s, 2H), 7.11 (d, 1H), 7.31 (d, 1H), 7.35 (d, 1H), 8.00 (dd, 1H), 9.06 (d, 1H).

### EXEMPLE 19 :

### Acide 3-(4-Méthoxyéthoxyméthoxy-5,5,8,8-tétraméthyl-5,6,7,8-tétrahydronaphthalen-2-ylselanyl)-benzoïque

Le produit est obtenu de manière analogue à l'exemple 7, à partir de 4-méthoxyéthoxyméthoxy-5,6,7,8-tetrahydro-5,5,8,8-tétramethylnaphtalene-2-disélénide et du 3-iodo-benzoate d'éthyle.
RMN ¹H/CDCl₃:1.26 (s, 6H); 1.38 (s, 6H), 1.62 (m, 4H), 3.36 (s, 3H), 3.53 (m, 2H), 3.78 (m, 2H), 5.22 (s, 2H), 7.12 (d, 1H), 7.15 (d, 1H), 7.35 (t, 1H), 7.65 (dd, 1H), 7.96 (dd, 1H), 8.20 (d, 1H).

### EXEMPLE 20 :

### Acide 4-(4-Méthoxyéthoxyméthoxy-5,5,8,8-tétraméthyl-5,6,7,8-tétrahydronaphthalen-2-ylselanyl)-3-méthoxy-benzoïque .

Le produit est obtenu de manière analogue à l'exemple 7, à partir de 4-méthoxyéthoxyméthoxy-5,6,7,8-tetrahydro-5,5,8,8-tétramethylnaphtalene-2-disélénide et du 4-iodo-3-méthoxybenzoate d'éthyle.
RMN ¹H/CDCl₃:1.26 (s, 6H); 1.42 (s, 6H), 1.66 (m, 4H), 3.35 (s, 3H), 3.54 (m, 2H), 3.81 (m, 2H), 3.98 (s, 3H), 5.27 (s, 2H), 6.94 (d, 1H), 7.25 (d, 1H), 7.30 (d, 1H), 7.48 à 7.53 (m, 2H).

### EXEMPLE 21 :

### Acide 3-(4-Méthoxyéthoxyméthoxy-5,5,8,8-tétraméthyl-5,6,7,8-tétrahydronaphthalen-2-ylselanyl)-4-méthoxy-benzoïque

Le produit est obtenu de manière analogue à l'exemple 7, à partir de 4-méthoxyéthoxyméthoxy-5,6,7,8-tetrahydro-5,5,8,8-tétramethylnaphtalene-2-disélénide et du 3-iodo-4-méthoxybenzoate d'éthyle.
RMN ¹H/CDCl₃:1.25 (s, 6H); 1.40 (s, 6H), 1.65 (m, 4H), 3.34 (s, 3H), 3.53 (m, 2H), 3.80 (m, 2H), 3.97 (s, 3H), 5.26 (s, 2H), 6.88 (d, 1H), 7.21 (d, 1H), 7.24 (d, 1H), 7.82 (d, 1H), 7.94 (dd, 1H).

### EXEMPLE 22 :

### Acide 6-(4-Méthoxyméthoxy-5,5,8,8-tétraméthyl-5,6,7,8-tétrahydronaphthalen-2-ylselanyl)-nicotinique

a) 4-méthoxyméthoxy-5,6,7,8-tétrahydro-5,5,8,8-tétramethylnaphtalene-2-disélénide
   De manière analogue à l'exemple 1(a), à partir du 2-bromo-5,5,8,8-tetramethyl-4-methoxyméthoxy-5,6,7,8-tetrahydro-naphthalene, on obtient le composé attendu sous forme d'une huile orange.
b) Acide 6-(4-Méthoxyméthoxy-5,5,8,8-tétraméthyl-5,6,7,8-tétrahydronaphthalen-2-ylselanyl)-nicotinique
   Le produit est obtenu de manière analogue à l'exemple 7, à partir de 4-méthoxyméthoxy-5,6,1,8-tetrahydro-5,5,8,8-tétramethylnaphtalene-2-disélénide et du 6-iodo-nicotinate d'éthyle.
   RMN ¹H/CDCl₃:1.27 (s, 6H); 1.43 (s, 6H), 1.67 (m, 4H), 3.49 (s, 3H), 5.20 (s, 2H), 7.11 (d, 1H), 7.24 (d, 1H), 7.35 (d, 1H), 8.01 (dd, 1H), 9.07 (d, 1H).

### EXEMPLE 23 :

### Acide 6-(3-Méthoxyéthoxyméthoxy-5,5,8,8-tétraméthyl-5,6,7,8-tétrahydronaphthalen-2-ylselanyl)-nicotinique

a) 3-méthoxyéthoxyméthoxy-5,6,7,8-tetrahydro-5,5,8,8-tetramethylnaphtalene-2-disélénide
   De manière analogue à l'exemple 1(a), à partir du 2-bromo-5,5,8,8-tetramethyl-3-methoxyéthoxymethoxy-5,6,7,8-tetrahydro-naphthalen, on obtient le composé attendu sous forme d'une huile orange.
b) Acide 6-(3-Méthoxyéthoxyméthoxy-5,5,8,8-tétraméthyl-5,6,7,8-tétrahydronaphthalen-2-ylselanyl)-nicotinique
   Le produit est obtenu de manière analogue à l'exemple 7, à partir de 3-méthoxyéthoxyméthoxy-5,6,7,8-tetrahydro-5,5,8,8-tétramethylnaphtalene-2-disélénide et du 6-iodo-nicotinate d'éthyle.
   RMN ¹H/CDCl₃:1.25 (s, 6H); 1.31 (s, 6H), 1.69 (s, 4H), 3.36 (s, 3H), 3.51 (m, 2H), 3.74 (m, 2H), 5.22 (s, 2H), 7.04 (d, 1H), 7.23 (s, 1H), 7.61 (s, 1H), 7.97 (dd, 1H), 9.05 (d, 1H).

### EXEMPLE 24 :

### Acide 2-(3-Méthoxyéthoxyméthoxy-5,5,8,8-tétraméthyl-5,6,7,8-tétrahydronaphthalen-2-ylselanyl)-nicotinique

Le produit est obtenu de manière analogue à l'exemple 7, à partir de 3-méthoxyéthoxyméthoxy-5,6,7,8-tetrahydro-5,5,8,8-tétramethylnaphtalene-2-disélénide et du 2-iodo-nicotinate d'éthyle.
RMN ¹H/CDCl₃:1.25 (s, 6H); 1.31 (s, 6H), 1.68 (s, 4H), 3.37 (s, 3H), 3.52 (m, 2H), 3.74 (m, 2H), 5.17 (s, 2H), 7.10 (dd, 1H), 7.22 (s, 1H), 7.54 (s, 1H), 8.29 (dd, 1H), 8.44 (dd, 1H).

### EXEMPLE 25 :

### Acide 4-(3-Méthoxyéthoxyméthoxy-5,5,8,8-tétraméthyl-5,6,7,8-tétrahydronaphthalen-2-ylselanyl)-benzoïque

Le produit est obtenu de manière analogue à l'exemple 7, à partir de 3-méthoxyéthoxyméthoxy-5,6,7,8-tetrahydro-5,5,8,8-tétramethylnaphtalene-2-disélénide et du 4-iodo-benzoate d'éthyle.
RMN ¹H/CDCl₃:1.19 (s, 6H); 1.29 (s, 6H), 1.63 (s, 4H), 3.36 (s, 3H), 3.50 (m, 2H), 3.71 (m, 2H), 5.22 (s, 2H), 7.16 (s, 1H), 7.36 (s, 1H), 7.41 (d, 2H), 7.93 (d, 2H).

### EXEMPLE 26 :

### Acide 3-(3-Méthoxyéthoxyméthoxy-5,5,8,8-tétraméthyl-5,6,7,8-tétrahydronaphthalen-2-ylselanyl)-benzoïque

Le produit est obtenu de manière analogue à l'exemple 7, à partir de 3-méthoxyéthoxyméthoxy-5,6,7,8-tetrahydro-5,5,8,8-tétramethylnaphtalene-2-disélénide et du 3-iodo-benzoate d'éthyle.
RMN ¹H/CDCl₃:1.12 (s, 6H); 1.27 (s, 6H), 1.63 (m, 4H), 3.37 (s, 3H), 3.52 (m, 2H), 3.77 (m, 2H), 5.26 (s, 2H), 7.12 (s, 1H), 7.13 (s, 1H), 7.38 (t, 1H), 7.69 (dd, 1H), 7.99 (dd, 2H), 8.25 (d, 1H).

### EXEMPLE 27 :

### Acide 6-(3,5-Di-tert-butyl-2-méthoxyméthoxy-phénylselanyl)-nicotinique

a) 2-bromo-4,6-di-*tert*-butyl-1-méthoxyméthoxyphenyl.
   Un mélange de 2-bromo-4,6-di-*tert*-butyl-phénol (4,4 mmol), de carbonate de césium (2,95 g) et de chlorure de méthoxyméthyle (4,8 mmol) dans le DMF (18 ml) est agité à température ambiante pendant 24 h. Le milieu réactionnel est extrait par de l'éther éthylique. La phase organique est lavée à l'eau, séchée sur sulfate de magnésium et concentrée à l'évaporateur rotatif. Le produit est purifié par filtration sur silice
b) 4,6-di-tert-butyl-1-méthoxyméthoxyphen-2-yl diselenide.
   De manière analogue à l'exemple 10(c), à partir de 10 g du produit obtenu précédemment, de 1,1 g de magnésium et de 2,63 g de sélénium, on obtient 7,6 g (76%) du produit attendu sous forme d'un solide jaune.
   RMN ¹H/CDCl₃: 1.18 (s, 9H); 1.42 (s, 9H); 3.68 (s, 3H); 5.08 (s,2H); 7.23 (d,1H); 7.54 (d, 1H).
c) Acide 6-(3,5-Di-*tert*-butyl-2-méthoxyméthoxy-phénylselanyl)-nicotinique
   Le produit est obtenu de manière analogue à l'exemple 7, à partir de 4,6-di-tert-butyl-1-méthoxyméthoxyphen-2-yl disélénide et de 6-iodo-nicotinate d'éthyle
   RMN ¹H/CDCl₃: 1.30 (s, 9H); 1.45 (s, 9H); 3.51 (s, 3H); 5.17 (s,2H); 6.94 (d, 1H); 7.50 (d, 1H), 7.56 (d, 1H), 7.98 (dd, 1H), 9.05 (d, 1H).

### EXEMPLE 28:

### Acide 2-(3,5-Di-tert-butyl-2-méthoxyméthoxy-phénylselanyl)-nicotinique

Le produit est obtenu de manière analogue à l'exemple 7, à partir de 4,6-di-tert-butyl-1-méthoxyméthoxyphen-2-yl disélénide et de 2-iodo-nicotinate d'éthyle
RMN ¹H/CDCl₃: 1.30 (s, 9H); 1.46 (s, 9H); 3.51 (s, 3H); 5.16 (s,2H); 7.12 (dd, 1H); 7.44 (s, 1H), 7.44 (s, 1H), 8.30 (dd, 1H), 8.46 (dd, 1H).

### EXEMPLE 29 :

### Acide 4-(3,5-Di-tert-butyl-2-méthoxyméthoxy-phénylselanyl)-benzoïque

Le produit est obtenu de manière analogue à l'exemple 7, à partir de 4,6-di-tert-butyl-1-méthoxyméthoxyphen-2-yl disélénide et de 4-iodo-benzoate d'éthyle
RMN ¹H/CDCl₃: 1.25 (s, 9H); 1.44 (s, 9H); 3.55 (s, 3H); 5.15 (s,2H); 7.33 à 7.41 (m, 3H); 7.92 (d, 2H).

### EXEMPLE 30 :

### Acide 3-(3,5-Di-tert-butyl-2-méthoxyméthoxy-phénylselanyl)-benzoïque

Le produit est obtenu de manière analogue à l'exemple 7, à partir de 4,6-di-tert-butyl-1-méthoxyméthoxyphen-2-yl disélénide et de 3-iodo-benzoate d'éthyle
RMN ¹H/CDCl₃: 1.20 (s, 9H); 1.44 (s, 9H); 3.60 (s, 3H); 5.17 (s,2H); 7.15 (d, 1H), 7.32 à 7.36 (m, 2H), 7.56 (dd, 1H); 7.96 (dd, 1H), 8.18 (d, 1H).

### EXEMPLE 31 :

### Acide 6-[4-adamantan-1-yl-3-benzyloxy-phenylsélenalyl]-nicotinique

a) 2-(adamantan-1-yl)-5-bromo-1-(2-méthoxyéthoxyméthoxy)-phenyl
   De l'hydrure de sodium à 60% (2,5 g) est additionné par fractions à une solution de 2-(adamantan-1-yl)-5-bromo-1-phenol (20,9 g) dans un mélange de THF et de DMF (5/5). L'agitation est poursuivie 30 mn à T.A. après la fin de l'addition, puis du chlorure de méthoxyéthoxyméthyle (8,92g) est additionné. Le milieu réactionnel est agité 4 h. à T.A. puis est traité avec de l'eau et de l'éther éthylique. La phase organique est lavée à l'eau, séchée sur sulfate de magnésium et concentrée. Après filtration sur silice, on obtient 17 g (64%) de produit attendu sous forme d'un solide blanc. Pf=88°C.
b) 4-Adamantan-1-yl-3-(2-methoxy-ethoxymethoxy)-phenyldisélénide.
   De manière analogue à l'exemple 1 (a), à partir de 13,04 g de 2-(adamantan-1-yl)-5-bromo-1-méthoxyéthoxyméthoxyphenyl, on obtient 9,9 g (76%) de produit attendu sous forme d'une huile jaune.
   RMN 1H/CDCl3: 1.55 (s, 6H) ; 2.05 (d,9H); 3.38 (s,3H); 3.57 (m, 2H) ; 3.82 (m, 2H) ; 5.27 (s, 2H), 7,11 (d, 1H) ; 7.22 (dd, 1H) ; 7.38 (d, 1H).
c) 4-Adamantan-1-yl-3-hydroxy-phenyldisélénide.
   Un mélange du produit précédemment obtenu (200 mg), d'acide sulfurique concentré (1,4 ml) de méthanol (20ml) et de THF (20 ml) est agité 12h à température ambiante. Le milieu réactionnel est extrait à l'acétate d'éthyle. La phase organique est lavée deux fois à l'eau séchée sur sulfate de magnésium et concentré à l'évaporateur rotatif sous vide. Le produit attendu est purifié par chromatographie flash pour donner une poudre orangée.
d) 4-Adamantan-1-yl-3-benzyloxy-phenyldisélénide.
   Un mélange du produit précédemment obtenu (4,4 mmol), de carbonate de césium (2,95 g) et de chlorure de benzyle (1,3 ml) dans le DMF (18 ml) est agité à température ambiante pendant 24 h. Le milieu réactionnel est extrait par de l'éther éthylique. La phase organique est lavée à l'eau, séchée sur sulfate de magnésium et concentrée à l'évaporateur rotatif. Le produit est purifié par filtration sur silice (heptane puis dichlorométhane). On obtient le composé attendu sous forme d'une poudre jaune.
e) Acide 6-[4-adamantan-1-yl-3-benzyloxy-phenylsélenalyl]-nicotinique
   Le produit est obtenu de manière analogue à l'exemple 7, à partir de 4-Adamantan-1-yl-3-benzyloxy-phenyldisélénide et de 6-iodo-nicotinate d'éthyle
   RMN ¹H/ CDCl₃, acétone D₆: 1.74 (s, 6H); 2.06 (s, 3H); 2.17 (s, 6H); 5.12 (s,2H); 6.97 (d, 1H), 7.26 à 7.48 (m, 8H), 7.95 (dd, 1H), 9.04 (d, 1H).

### EXEMPLE 32 :

### Acide 6-(3,5-Di-tert-butyl-2-benzyloxy-phénylselanyl)-nicotinique

a) 3,5-Di-*tert*-butyl-2-benzyloxy-phényldisélénide
   Le mode opératoire est identique à celui suivi pour l'exemple 31 (c)et 31 (d), appliqué au produit de l'exemple 27 (b).
b) Acide 6-(3,5-Di-*tert*-butyl-2-benzyloxy-phénylselanyl)-nicotinique
   Le produit est obtenu de manière analogue à l'exemple 7, à partir de 3,5-Di-*tert*-butyl-2-benzyloxy-phényldisélénide et de 6-iodo-nicotinate d'éthyle
   ¹H/ CDCl₃ acétone D₆: 1.33 (s, 9H); 1.44 (s, 9H); 5.13 (s, 2H); 7.00 (d, 1H); 7.24 à 7.32 (m, 5H), 7.51 (d, 1H), 7.60 (d, 1H), 7.98 (dd, 1H), 9.01 (d, 1H).

### EXEMPLE 33 :

### Acide 3-méthoxy-4-(4-benzyloxy-5,6,7,8-tétrahydro-5,5,8,8-tétraméthyl naphtalen-2-ylsélanyl)-benzoïque

a) 4-hydroxy-5,6,7,8-tetrahydro-5,5,8,8-tetramethylnaphtalene-2-disélénide
   Le produit de l'exemple 22(a), 4-méthoxyméthoxy-5,6,7,8-tetrahydro-5,5,8,8-tetraméthylnaphtalene-2-disélénide (12,4g) est traité de manière analogue à l'exemple 15(b), on obtient 11 g (100%) du composé attendu sous forme d'un solide jaune. Tf=200°C.
   RMN ¹H/ CDCl₃: 1.22(s,6H); 1.42(s,6H); 1.63(m,4H); 5.25(s,1H); 6.75(d,1H); 7.11(d,1H)
b) 4-benzyloxy-5,6,7,8-tetrahydro-5,5,8,8-tetramethylnaphtalene-2-disélénide
   Un mélange du produit précédemment obtenu (2,5 g, 4,4 mmol), de carbonate de césium (2,95 g) et de chlorure de benzyle (1,3 ml) dans le DMF (18 ml) est agité à température ambiante pendant 24 h. Le milieu réactionnel est extrait par de l'éther éthylique. La phase organique est lavée à l'eau, séchée sur sulfate de magnésium et concentrée à l'évaporateur rotatif. Le produit est purifié par filtration sur silice (heptane puis dichlorométhane). On obtient 2,1 g (63%) du composé attendu sous forme d'une poudre jaune.
   RMN 1H/CDCl3: 1.21 (s,6H); 1.34(s,6H); 1.59 (m,4H); 4.96 (s,2H); 7.02 (d,1H); 7.21(d,1H); 7.29 à 7.41 (m, 5H).
c) Acide 3-méthoxy-4-(4-benzyloxy-5,6,7,8-tétrahydro-5,5,8,8-tétraméthyl naphtalen-2-ylsélanyl)-benzoïque
   Le produit est obtenu de manière analogue à l'exemple 7, à partir de 4-benzyloxy-5,6,7,8-tetrahydro-5,5,8,8-tetramethylnaphtalene-2-disélénide et de 4-iodo-3-méthoxybenzoate d'éthyle
   RMN ¹H/ CDCl₃: 1.27 (s, 6H), 1.43 (s, 6H), 1.66 (m,4H), 3.98 (s, 3H), 5.04 (s, 2H), 6.88 (d, 1H), 7.01 (d, 1H), 7.29 (s, 1H), 7.33 à 7.52 (m, 7H).

### EXEMPLE 34 :

### Acide 4-(4-benzyloxy-5,6,7,8-tétrahydro-5,5,8,8-tétraméthylnaphtalen-2-ylsélanyl)-benzoïque

Le produit est obtenu de manière analogue à l'exemple 7, à partir de 4-benzyloxy-5,6,7,8-tetrahydro-5,5,8,8-tetramethylnaphtalene-2-disélénide et de 4-iodo-benzoate d'éthyle
RMN ¹H/ CDCl₃: 1.26 (s. 6H), 1.41 (s, 6H), 1.65 (m, 4H), 5.02 (s. 2H), 6.92 (d. 1H), 7.22 (d. 1H), 7.31 à 7.41 (m. 7H),. 7.90 (d, 2H).

### EXEMPLE 35 :

### Acide 6-(4-benzyloxy-5,6,7,8-tétrahydro-5,5,8,8-tétraméthylnaphtalen-2-ylsélanyl)-nicotinique

Le produit est obtenu de manière analogue à l'exemple 7, à partir de 4-benzyloxy-5,6,7,8-tetrahydro-5,5,8,8-tetramethylnaphtalene-2-disélénide et de 6-iodo-nicotinate d'éthyle
RMN ¹H/CDCl₃: 1.28 (s. 6H), 1.43 (s, 6H), 1.67 (m, 4H), 5.07 (s, 2H), 7.00 (d, 1H), 7.04 (d, 1H), 7.32 à 7.44 (m, 6H), 7.96 (dd, 1H), 9.06 (d, 1H).

### EXEMPLE 36 :

### Acide 3-méthoxy-4-(3-benzyloxy-5,6,7,8-tétrahydro-5,5,8,8-tétraméthyl naphtaten-2-ylsélanyl)-benzoïque

a) 3-hydroxy-5,6,7,8-tetrahydro-5,5,8,8-tetramethylnaphtalene-2-disélénide
   Le produit de l'exemple 23(a), 3-méthoxyéthoxyméthoxy-5,6,7,8-tetrahydro-5,5,8,8-tetraméthylnaphtalene-2-disélénide est traité de manière analogue à l'exemple 31(c), on obtient le composé attendu sous forme d'un solide jaune (100%).
b) 3-benzyloxy-5,6,7,8-tetrahydro-5,5,8,8-tetramethylnaphtalene-2-disélénide
   Le produit précédemment obtenu est traité de manière analogue à l'exemple 33(b)
c) Acide 3-méthoxy-4-(3-benzyloxy-5,6,7,8-tétrahydro-5,5,8,8-tétraméthyl naphtalen-2-ylsélanyl)-benzoïque
   Le produit est obtenu de manière analogue à l'exemple 7, à partir de 3-benzyloxy-5,6,7,8-tetrahydro-5,5,8,8-tetramethylnaphtalene-2-disélénide et de 4-iodo-3-méthoxybenzoate d'éthyle
   RMN ¹H/ CDCl₃: 1.22 (s, 6H), 1.25 (s, 6H), 1.67 (s, 4H), 3.97 (s, 3H), 5.07 (s, 2H), 6.89 (d, 1H), 6.90 (d, 1H), 7.22 à 7.25 (m, 5H), 7.50 à 7.53 (m, 3H).

### EXEMPLE 37 :

### Acide 6-(3-benzyloxy-5,6,7,8-tétrahydro-5,5,8,8-tétraméthylnaphtalen-2-ylsélanyl)-nicotinique

Le produit est obtenu de manière analogue à l'exemple 7, à partir de 3-benzyloxy-5,6,7,8-tetrahydro-5,5,8,8-tetramethylnaphtalene-2-disélénide et de 6-iodo-nicotinate d'éthyle
RMN ¹H/ acétone D_{6,} CDCl₃: 1.25 (s, 6H), 1.27 (s, 6H), 1.68 (s, 4H), 5.08 (s. 2H), 6.94 (s, 1H), 7.04 (d, 1H), 7.31 (s, 3H), 7.62 (s, 1H), 7.94 (dd, 1H), 9.04 (d, 1H).

### EXEMPLE 38 :

### Acide 4-(3-hexyloxy-5,6,7,8-tétrahydro-5,5,8,8-tétraméthylnaphtalen-2-ylsélanyl)3-méthoxy-benzoïque

a) 3-hexyloxy-5,6,7,8-tetrahydro-5,5,8,8-tetramethylnaphtalene-2-disélénide
   De l'hydrure de sodium à 60% (225 mg, 5,63 mmol) est additionné par fractions à une solution de 4-hydroxy-5,6,7,8-tetrahydro-5,5,8,8-tetramethylnaphtalene-2-disélénide (1,2 g, 2,56 mmol) dans 15 ml de THF et 15 ml de THF. L'agitation est poursuivie 30 mn à T.A. après la fin de l'addition, puis du iodohexane (1 ml, 6,8 mmol) est additionné. Le milieu réactionnel est agité 4 h. à T.A. puis est traité avec de l'eau et de l'éther éthylique. La phase organique est lavée à l'eau, séchée sur sulfate de magnésium et concentrée. Après purification par chromatographie sur silice (heptane 95, CH2Cl2 5), le produit est obtenu sous forme d'une huile jaune.
   RMN ¹H/CDCl₃: 0.90 (m, 9H); 1.30 à 1.48 (m,12H); 1.59 (m,4H); 1.77 (m, 2H); 3.85 (t, 2H), 6.92(d, 1H); 7.17(d, 1H).
b) Acide 4-(3-hexyloxy-5,6,7,8-tétrahydro-5,5,8,8-tétraméthylnaphtalen-2-ylsélanyl)3-méthoxy-benzoïque
   Le produit est obtenu de manière analogue à l'exemple 7, à partir de 3-hexyloxy-5,6,7,8-tetrahydro-5,5,8,8-tetramethylnaphtalene-2-disélénide et de 4-iodo-3-méthoxybenzoate d'éthyle
   RMN ¹H/ CDCl₃: 0.89 (t. 3H), 1.27 (s, 6H), 1.30 à 1.37 (m, 4H), 1.42 (s, 6H), 1.48 (m, 2H), 1.63 (m. 4H), 1.82 (m. 2H), 3.90 (t, 2H), 3.98 (s, 3H), 6.91 (d, 1H), 6.93 (s, 1H), 7.24 (s, 1H), 7.49 à 7.55 (m, 2H).

### EXEMPLE 39 :

### Acide 6-(3-hexyloxy-5,6,7,8-tétrahydro-5,5,8,8-tétraméthylnaphtalen-2-ylsélanyl)-nicotinique

Le produit est obtenu de manière analogue à l'exemple 7, à partir de 3-hexyloxy-5,6,7,8-tetrahydro-5,5,8,8-tetramethylnaphtalene-2-disélénide et de 6-iodo-nicotinate d'éthyle
RMN ¹H/ CDCl₃: 0.89 (t, 3H), 1.27 (s, 6H), 1.30 à 1.37 (m, 4H), 1.42 (s, 6H), 1.48 (m, 2H). 1.63 (m, 4H), 1.84 (m, 2H), 3.92 (t, 2H), 6.97 (d, 1H), 7.08 (d. 1H), 7.29 (d, 1H), 8.00 (dd, 1H), 9.08 (d, 1H).

### EXEMPLE 40 :

### Acide 4-(5-adamantan-1-yl-4-benzyloxy-2-méthyl-phenylselenalyl)-benzoïque

a) 5-Adamantan-1-yl-4-benzyloxy-2-méthyl-phenyl disélénide.
   Le mode opératoire est identique à celui suivi pour l'exemple 31 (c)et 31 (d), appliqué au produit de l'exemple 16 (a).
b) Acide 4-(5-adamantan-1-yl-4-benzyloxy-2-méthyl-phenylselenalyl)-benzoïque
   Le produit est obtenu de manière analogue à l'exemple 7, à partir de 5-Adamantan-1-yl-4-benzyloxy-2-méthyl-phenyl disélénide et de 4-iodobenzoate d'éthyle.
   RMN ¹H/ acétone D_{6,} CDCl₃:1.70 (s, 6H); 2.02 (s, 3H), 2.11 (s, 6H), 2.41 (s, 3H), 5.16 (s, 2H), 6.85 (dd, 1H), 6.98 (s, 1H), 7.35 à 7.58 (m, 6H), 7.97 (dd, 2H), 9.05 (d, 1H).

### EXEMPLE 41 :

### 6-[3-[5-(tert-butyl-diméthyl-silanyloxy)-pentyloxyméthyl]-5,6,7,8-tétrahydro-5,5,8,8-tétraméthylnaphthalen-2-ylsélanyl] nicotinate d'éthyle.

a) Acétate de 5-(3-bromo-5,5,8,8-tétraméthyl-5,6,7,8-tétrahydro-naphthalen-2-yloxy)-pentyl.
   Une solution de 3-bromo-5,5,8,8-tétraméthyl-5,6,7,8-tétrahydronaphtalen-2-ol (10g, 0,35 mol), d'acétate de 5-bromopentyle (8,15 g), et de carbonate de potassium (33,6 g) dans la méthyléthylcétone (200 ml), est chauffée à reflux pendant 2 heures. Le milieu réactionnel est traité par de l'eau et de l'acétate d'éthyle. Après décantation, la phase organique est lavée deux fois à l'eau, séchée sur sulfate de magnésium et concentrée à l'évaporateur rotatif sous vide à 40 °C. Le produit est purifié par chromatographie flash sur colonne de silice.
   Huile jaune. Rendement: 93%.
b) [5-(3-Bromo-5,5,8,8-tétraméthyl-5,6,7,8-tétrahydro-naphthalen-2-yloxy)-pentyloxy]-*tert*-butyl-diméthyl-silane
   L'acétate précedement obtenu est saponifié puis le groupement hydroxyle résultant est protégé selon le mode opératoire suivant: Du chlorure de tert-butyldiméthylsilane (2,64 g) est additioné à un mélange de 5-(3-Bromo-5,5,8,8-tétraméthyl-5,6,7,8-tétrahydro-naphthalen-2-yloxy)-pentan-1-ol (4,3g, 11,7 mmol) et d'hydrure de sodium à 80% (422 mg) dans le THF (20ml).
   Le mélange est agité à température ambiante 2 h. La solution est versée dans un mélange d'eau et d'acétate d'éthyle. La phase organique est lavée deux fois par de l'eau, séchée sur sulfate de magnésium, et concentrée à l'évaporateur rotatif sous vide à 40°C. Le produit est purifié par chromatographie flash sur colonne de silice.
   Huile jaune. Rendement: 64%.
c) 3-[5-(*tert*-Butyl-dimethyl-silanyloxy)-pentyloxy]-5,5,8,8-tetramethyl-5,6,7,8-tetrahydro-naphthalene-2-disélénide
   Le produit attendu est obtenu à partir du bromé précédemment obtenu, de façon similaire à l'exemple 1a. Huile jaune. Rendement : 10%.
d) 6-[3-[5-(*tert*-butyl-diméthyl-silanyloxy)-pentyloxy]-5,6,7,8-tétrahydro-5,5,8,8-tétraméthylnaphthalen-2-ylsélanyl] nicotinate d'éthyle.
   De manière analogue à l'exemple 1(b), par réaction de 257 mg (0,27 mmol) de disélénide précédemment obtenu dans 25 ml d'éthanol avec 119 mg de borohydrure de sodium, 120 mg (0,43 mmol) de 6-iodonicotinate d'éthyle et 4 mg de dibromure de bis (bipyridine) nickel (II) , on obtient 152 mg (56%) du dérivé attendu sous forme d'une huile jaune.
   RMN 1H (CDCl₃) : 0,00 (6H, s), 0,85 (9H, s), 1,22 (6H, s), 1,30 (6H, s), 1,33 à 1,50 (6H, m), 1,60 à 1,67 (7H, m), 3,48 (2H, t), 3,92 (2H, t), 4,35 (2H, q), 6,84 (1H, s),. 6,99 (1H, d), 7,57 (1H,s), 7,91 (1H, dd), 8,97 (1H, d).

### EXEMPLE 42 :

### Acide 6-[3-[5-(tert-butyl-diméthyl-sitanyloxy)-pentyloxy]-5,6,7,8-tétrahydro-5,5,8,8-tétraméthylnaphthalen-2-ylsélanyl] nicotinique.

De manière analogue à l'exemple 2, par réaction de 312 mg (0,49 mmol) de 6-[3-[5-(*tert*-butyl-diméthyl-silanyloxy)-pentyloxy]-5,6,7,8-tétrahydro-5,5,8,8-tétraméthylnaphthalen-2-ylsélanyl] nicotinate d'éthyle avec 213 mg (5,3 mmol) de soude dans un mélange THF/éthanol (5ml/5ml), on obtient 210 mg (71%) de poudre jaune. Tf :161 °C.

### EXEMPLE 43 :

### Acide 6-[3-(5-hydroxypentyloxy)-5,6,7,8-tétrahydro-5,5,8,8-tétraméthyl naphthalen-2-ylsélanyl] nicotinique.

Un mélange du produit de l'exemple précédent (210 mg, 0,35 mmol), d'une solution 1M dans le THF de fluorure de tétra-n-butylammonium (380 ul) dans le THF 5ml) est agité à température ambiante 3h. 380 ul de la solution de fluorure de tétra-n-butylammonium sont rajoutés au milieu réactionnel. L'agitation est poursuivie 3,5 h, puis on rajoute encore 380 ul de TBAF et l'addition est poursuivie 1 h20 de plus. Le milieu réactionnel est traité par une solution d'HCl 1N et de l'acétate d'éthyle. Après décantation la phase organique est lavée à l'eau, séchée sur sulfate de magnésium anhydre et concentrée. Le produit est purifié par cristallisation dans un mélange heptane, éther éthylique. Masse : 194 mg, poudre blanche. Pf=190-192°C.

### EXEMPLE 44 :

### 4-(5,5,8,8-Tétraméthyl-5,6,7,8-tetrahydro-naphthalen-2-ylselanyl)-benzoate d'ethyle.

(a) 5,6,7,8-tetrahydro-5,5,8,8-tetramethylnaphtalene-2-disélénide.
   Une solution de tert-butyllithium 1,7 M dans le pentane (37,4 mmol, 22 ml) est additionnée à une solution de 2-bromo-5,6,7,8-tetrahydro-5,5,8,8-tetraméthylnaphtalene (4,22 g, 15,8 mmol) dans le THF (100 ml) à -78°C en 10 min. Le mélange est agité à 0°C 30 min. Le sélénium(1,33 g, 16,8 mmol) est additionné en 2 fois. Le mélange est agité à 0°C 15 min, puis à température ambiante 30 min. Une solution d'HCl 1N (40 ml) est additionnée, puis le mélange réactionnel est traité par de l'éther éthylique. La phase organique est lavée 2 fois à l'eau, séchée sur sulfate de magnésium anhydre et concentrée à l'évaporateur rotatif sous vide à 40°C. 10 ml d'éthanol et 50 mg de soude sont additionnés à l'huile obtenue. Le mélange est agité vigoureusement quelques minutes à l'air (qsp tout précipite), puis est concentré à l'évaporateur rotatif sous vide à 40°C. Le solide obtenu est filtré sur silice (élution heptane) puis cristallisé dans un mélange éthanol / éther.
   Solide orange. Masse : 2,9 g. Rendement : 69%.
   RMN 1H (CDCl₃): 1,21 (6H, s), 1,25 (6H, s), 1,65 (4H, s), 7,20 (1H Ar, d, J=8,25 Hz), 7,38 (1H Ar, dd ,J=1,9 Hz, J=8,25 Hz), 7,51 (1H Ar, d, J=1,9 Hz).
b) 4-(5,5,8,8-Tétraméthyl-5,6,7,8-tetrahydro-naphthalen-2-ylselanyl)-benzoate d'ethyle.
   De manière analogue à l'exemple 1(b), par réaction de 213 mg (0,4 mmol) de disélénide précédemment obtenu dans 20 ml d'éthanol avec 73 mg de borohydrure de sodium (1,92 mmol), 177 mg (0,64 mmol) de 4-iodobenzoate d'éthyle et 37 mg de tétrakis triphénylphosphine palladium. Après purification par chromatographie flash (heptane 70-CH₂Cl₂ 30), on obtient 151 mg du dérivé attendu sous forme d'un solide jaune. Tf=73°C.
   RMN 1H (CDCl₃) : 1,26 (6H, s), 1,29 (6H, s), 1,37 (t, 3H), 1,70 (4H, s), 4,34 (q, 2H), 7,15 à 7,25 (m, 3H), 7,32 (1H, d), 7,44 (1H, d), 7,89 (1H, d).

### EXEMPLE 45 :

### 4-(3-Méthoxyéthoxyméthoxy-5,5,8,8-tétraméthyl-5,6,7,8-tétrahydronaphthalen-2-ylselanyl)-benzoate d'éthyle

De manière analogue à l'exemple 1(b), par réaction de 3,35 g (4,5 mmol) de 3-méthoxyéthoxyméthoxy-5,6,7,8-tetrahydro-5,5,8,8-tetramethylnaphtalene-2-disélénide dans 100 ml d'éthanol avec 501 mg de borohydrure de sodium (13,5 mmol), 2,5 g (9 mmol) de 4-iodobenzoate d'éthyle et 90 mg de Bis (bipyridine) nickel II dibromide. Après purification par chromatographie flash (heptane 85, AcOEt 15), on obtient 2,58 g du dérivé attendu sous forme d'une huile jaune (83%).

### EXEMPLE 46 :

### 4-(3-Hydroxy-5,5,8,8-tétraméthyl-5,6,7,8-tétrahydro-naphthalen-2-ylselanyl)-benzoate d'éthyle.

Un mélange de 4-(3-Méthoxyéthoxyméthoxy-5,5,8,8-tétraméthyl-5,6,7,8-tétrahydro-naphthalen-2-ylselanyl)-benzoate d'éthyle (2,3 g, 4,4 mmol) d'acide sulfurique concentré (475 ul) de méthanol (40ml) et de THF (20 ml) est agité 48h. à température ambiante. Le milieu réactionnel est extrait à l'éther d'éthylique. La phase organique est lavée deux fois à l'eau séchée sur sulfate de magnésium et concentré à l'évaporateur rotatif sous vide. Le produit est purifié par cristallisation dans l'heptane. On obtient 2,06 g (97%) du composé attendu sous forme d'une poudre orangée. Tf=113°C.

### EXEMPLE 47 :

### Acide 4-(3-Hydroxy-5,5,8,8-tétraméthyl-5,6,7,8-tétrahydro-naphthalen-2-ylselanyl)-benzoïque.

De manière analogue à l'exemple 2, par réaction de 400 mg (0,92 mmol) de 4-(3-Hydroxy-5,5,8,8-tétraméthyl-5,6,7,8-tétrahydro-naphthalen-2-ylselanyl)-benzoate d'éthyle avec 336 mg (8,4 mmol) de soude dans un mélange THF/éthanol (20ml/20ml), on obtient 214 mg (58%) de poudre rose. Tf :217°C.

### EXEMPLE 48 :

### 6-(3-Méthoxyéthoxyméthoxy-5,5,8,8-tétraméthyl-5,6,7,8-tétrahydronaphthalen-2-ylselanyl)-nicotinate d'éthyle

De manière analogue à l'exemple 1(b), par réaction de 3,35 g (4,5 mmol) de 3-méthoxyéthoxyméthoxy-5,6,7,8-tetrahydro-5,5,8,8-tetramethylnaphtalene-2-disélénide dans 100 ml d'éthanol avec 501 mg de borohydrure de sodium (13,5 mmol), 2,5 g (9 mmol) de 4-iodobenzoate d'éthyle et 90 mg de Bis (bipyridine) nickel II dibromide. Après purification par chromatographie flash (heptane 85, AcOEt 15), on obtient 2,09 g du dérivé attendu sous forme d'une huile jaune (45%).
RMN ¹H/CDCl₃: 1,25 (s, 6H), 1,31 (s, 6H), 1,38 (t, 3H), 1,69 (m, 4H), 3,36 (s, 3H), 3,50 (m, 2H), 3,73 (m, 2H), 4,37 (q, 2H), 5,22 (s, 2H), 7,01 (d, 1H), 7,22 (s, 1H), 7,60 (s, 1H), 7,94 (dd, 1H), 8,99 (d, 1H).

### EXEMPLE 49 :

### 6-(3-Hydroxy-5,5,8,8-tétraméthyl-5,6,7,8-tétrahydro-naphthalen-2-ylselanyl)-nicotinate d'éthyle.

Un mélange de 6-(3-Méthoxyéthoxyméthoxy-5,5,8,8-tétraméthyl-5,6,7,8-tétrahydro-naphthalen-2-ylselanyl)-nicotinate d'éthyle (2.6 g, 5 mmol) d'acide sulfurique concentré (535 ul) d'éthanol (75ml) et de THF (25 ml) est agité 3 jours à température ambiante. Le milieu réactionnel est extrait à l'éther d'éthylique. La phase organique est lavée deux fois à l'eau séchée sur sulfate de magnésium et concentré à l'évaporateur rotatif sous vide. Le solide obtenu est lavé à l'éther éthylique. On obtient 2,01 g (93%) du composé attendu sous forme d'une poudre orangée. Tf=138°C.

### EXEMPLE 50 :

### Acide 6-(3-Hydroxy-5,5,8,8-tétraméthyl-5,6,7,8-tétrahydro-naphthalen-2-ylselanyl)-nicotinique.

De manière analogue à l'exemple 2, par réaction de 400 mg (0,92 mmol) de 6-(3-Hydroxy-5,5,8,8-tétraméthyl-5,6,7,8-tétrahydro-naphthalen-2-ylselanyl)-nicotinate d'éthyle avec 357 mg (8,9 mmol) de soude dans un mélange THF/éthanol (20ml/20ml), on obtient 60 mg (16%) de poudre jaune. Tf :250°C.

### EXEMPLE 51 :

### 6-[3-(3-éthoxy-carbonyl-propoxy)-5,5,8,8-tétraméthyl-5,6,7,8-tétrahydronaphthalen-2-ylselanyl]-nicotinate d'éthyle.

Dans un tricol, on introduit 432 mg (102,0 mmoles) de 6-(3-Hydroxy-5,5,8,8-tétraméthyl-5,6,7,8-tétrahydro-naphthalen-2-ylselanyl)-nicotinate d'éthyle, 276 mg (2 mmoles) de carbonate de potassium et 390 mg (2 mmol) de 4-bromobutanoate d'éthyle. Le mélange est chauffé à 80°C pendant 12h. On verse le milieu réactionnel dans l'eau, extrait avec de l'éther éthylique, décante la phase organique, lave à l'eau, sèche sur sulfate de magnésium, évapore. Après purification par chromatographie flash (heptane 9, AcOEt 1) , on recueille 467 mg (85%) du composé attendu, sous la forme d'une huile orangée.
RMN ¹H/CDCl₃: 1,20 à 1,31 (m, 15H), 1,38 (t, 3H), 1,69 (s, 4H), 1.96 (m. 2H), 2,38 (t, 2H), 2,85 (t, 2H), 4,12 (q, 2H), 4,36 (q, 2H), 6,88 (d, 1H), 7.02 (s, 1H), 7,48 (s, 1H), 8,26 (dd, 2H), 8,83 (d, 1H).

### EXEMPLE 52 :

### Acide 6-[3-(3-carboxy-propoxy)-5,5,8,8-tétraméthyl-5,6,7,8-tétrahydronaphthalen-2-ylselanyl]-nicotinique.

De manière analogue à l'exemple 2, par réaction de 340 mg (0,62 mmol) de 6-[3-(3-éthoxy-carbonyl-propoxy)-5,5,8,8-tétraméthyl-5,6,7,8-tétrahydronaphthalen-2-ylselanyl]-nicotinate d'éthyle.avec 250 mg (62,2 mmol) de soude dans l'éthanol (10ml), on obtient 211 mg (69%) de poudre blanche. Tf :177°C.

### EXEMPLE 53 :

### 4-[3-(3-ethoxycarbonyl-propoxy)-5,5,8,8-tétraméthyl-5,6,7,8-tétrahydronaphthalen-2-ylselanyl]-benzoate d'éthyle.

De manière analogue à l'exemple 51, par réaction de 300 mg (0,86 mmol) de 4-(3-hydroxy-5,5,8,8-tétraméthyl-5,6,7,8-tétrahydro-naphthalen-2-ylselanyl]-benzoate d'éthyle avec 336 mg (1,72 mmol) de 4-bromobutanoate d'éthyle et de 238 mg de carbonate de potassium dans la MEC (10ml), on obtient 364 mg (78%) d'une huile jaune.
RMN ¹H/CDCl₃: 1,16 à 1,32 (m, 15H), 1,38 (t, 3H), 1,66 (m, 4H), 1,98 (m, 2H), 2,30 (t, 2H), 3,98 (t, 2H), 4,08 (q, 2H), 4,35 (q, 2H), 6,78 (s, 1H), 7,28 (s, 1H), 7,41 (dd, 2H), 7,87 (dd, 2H).

### EXEMPLE 54 :

### Acide 4-[3-(3-carboxy-propoxy)-5,5,8,8-tétraméthyl-5,6,7,8-tétrahydronaphthalen-2-ylselanyl]-benzoïque.

De manière analogue à l'exemple 2, par réaction de 250 mg (0,46 mmol) de 4-[3-(3-carboxy-propoxy)-5,5,8,8-tétraméthyl-5,6,7,8-tétrahydro-naphthalen-2-ylselanyl]-benzoate d'éthyle avec 183 mg (4,6 mmol) de soude dans un mélange THF/éthanol (5/5ml), on obtient 172 mg (76%) de poudre blanche. Tf :230°C.

### EXEMPLE 55 :

### 4-[3-(7-méthoxycarbonyl-heptyloxy)-5,5,8,8-tétraméthyl-5,6,7,8-tétrahydronaphthalen-2-ylselanyl]-benzoate d'éthyle.

De manière analogue à l'exemple 51, par réaction de 370 mg (0,86 mmol) de 4-(3-hydroxy-5,5,8,8-tétraméthyl-5,6,7,8-tétrahydro-naphthalen-2-ylselanylbenzoate d'éthyle avec 408 mg (1,72 mmol) de 8-bromo-octanoate de méthyle et de 238 mg de carbonate de potassium dans la MEC (10ml), on obtient 502 mg (99%) d'une huile jaune.
RMN ¹H/CDCl₃: 1,16 (s, 6H), 1,26 à 1,29 (m, 12H), 1,38 (t, 3H), 1,56 à 1,68 (m, 8H), 2,28 (t, 2H), 3,66 (s, 3H), 3,92 (t, 2H), 4,36 (q, 2H), 6,78 (s, 1H), 7,27 (s, 1H), 7,41 (dd, 2H), 7,88 (dd, 2H).

### EXEMPLE 56 :

### Acide 4-[3-(7-carboxy-heptyloxy)-5,5,8,8-tétraméthyl-5,6,7,8-tétrahydronaphthalen-2-ylselanyl]-benzoïque.

De manière analogue à l'exemple 2, par réaction de 410 mg (0,7 mmol) de 4-[3-(7-méthoxycarbonyl-heptyloxy)-5,5,8,8-tétraméthyl-5,6,7,8-tétrahydronaphthalen-2-ylselanyl]-benzoate d'éthyle avec 280 mg (7 mmol) de soude dans un mélange THF/éthanol (5/5ml), on obtient 326 mg (85%) de poudre blanche. Tf:183°C.

### EXEMPLE 57 :

### 6-[3-(7-méthoxycarbonyl-heptyloxy)-5,5,8,8-tétraméthyl-5,6,7,8-tétrahydronaphthalen-2-ylselanyl]-nicotinate d'éthyle.

De manière analogue à l'exemple 51, par réaction de 460 mg (1,06 mmol) de 6-(3-hydroxy-5,5,8,8-tétraméthyl-5,6,7,8-tétrahydro-napththalen-2-ylselanylnicotinate d'éthyle avec 515 mg (2,17 mmol) de 8-bromo-octanoate de méthyle et de 295 mg de carbonate de potassium dans la MEC (10ml), on obtient 487 mg (78%) d'une huile jaune.

### EXEMPLE 58 :

### 6-[3-(7-carboxy-heptyloxy)-5,5,8,8-tétraméthyl-5,6,7,8-tétrahydronaphthalen-2-ylselanyl]-nicotinique.

De manière analogue à l'exemple 2, par réaction de 390 mg (0,66 mmol) de 6-[3-(7-méthoxycarbonyl-heptyloxy)-5,5,8,8-tétraméthyl-5,6,7,8-tétrahydronaphthalen-2-ylselanyl]-nicotinate d'éthyle avec 265 mg (6,6 mmol) de soude dans un mélange THF/éthanol (5/1ml), on obtient 277 mg (77%) de poudre blanche. Tf :186°C.

### EXEMPLE 59 :

### 6-(3-(2-acétoxy-éthoxy)-5,5,8,8-tétraméthyl-5,6,7,8-tétrahydro-naphthalen-2-ylselanyl)-nicotinate d'éthyle

a) acétate de (2-bromoéthyle).
   De l'anhydride acétique (11,35 ml, 0,12 mol) est additionné goutte à goutte à une solution de 2-bromoéthanol (12,5g, 0,1 mol), de DMAP (1,22g), dans 125 ml de dichlorométhane. Le mélange est agité à température ambiante 12h., traité à l'eau et au dichlorométhane. La phase organique est lavée à l'eau, séchée sur sulfate de magnésium et concentrée à l'évaporateur rotatif et purifié par distillation. Liquide jaunâtre (94%).
b) 6-(3-(2-acétoxy-éthoxy)-5,5,8,8-tétraméthyl-5,6,7,8-tétrahydro-naphthalen-2-ylselanyl)-nicotinate d'éthyle
   De manière analogue à l'exemple 51, par réaction de 477 mg (1,10 mmol) de 6-(3-hydroxy-5,5,8,8-tétraméthyl-5,6,7,8-tétrahydro-naphthalen-2-ylselanylnicotinate d'éthyle avec 396 mg (2,2 mmol) d'acétate de (2-bromoéthyle) et de 304 mg de carbonate de potassium dans la MEC (10ml), on obtient 545 mg (96%) d'une huile jaune.
   RMN ¹H/CDCl₃; 1.24 (s. 6H), 1,32 (s, 6H), 1.38 (t. 3H), 1,69 (s, 4H), 1,99 (s, 3H), 3,00 (t, 3H), 4,24 (t, 2H), 4,38 (q, 2H), 6,89 (d. 1H), 7,03 (s, 1H), 7,54 (s, 1H), 8,27 (dd, 1H), 8,83 (d, 1H).

### EXEMPLE 60 :

### Acide 6-(3-(2-hydroxy-éthoxy)-5,5,8,8-tétraméthyl-5,6,7,8-tétrahydronaphthalen-2-ylselanyl)-nicotinique

De manière analogue à l'exemple 2, par réaction de 419 mg (0,81 mmol) de 6-[3-(2-acétoxy-éthoxy)-5,5,8,8-tétraméthyl-5,6,7,8-tétrahydro-naphthalen-2-ylselanyl]-nicotinate d'éthyle avec 320 mg (8 mmol) de soude dans un mélange THF/éthanol (4/4ml), on obtient 273 mg (75%) de poudre blanche. Tf :170°C.

### EXEMPLE 61 :

### 4-(3-(2-acétoxy-éthoxy)-5,5,8,8-tétraméthyl-5,6,7,8-tétrahydro-naphthalen-2-ylselanyl)-benzoate d'éthyle

De manière analogue à l'exemple 51, par réaction de 400 mg (0,93 mmol) de 4-(3-hydroxy-5,5,8,8-tétraméthyl-5,6,7,8-tétrahydro-naphthalen-2-ylselanylbenzoate d'éthyle avec 334 mg (2,2 mmol) d'acétate de (2-bromoéthyle) et de 257 mg de carbonate de potassium dans la MEC (10ml), on obtient 333 mg (69%) d'une huile jaune.
RMN ¹H/CDCl₃: 1,16 (s, 6H), 1,29 (s, 6H), 1,38 (t, 3H), 1,59 (s, 4H), 1,99 (s, 3H), 4,16 (m, 2H), 4,29 à 4,40 (m, 4H), 6,82 (s, 1H), 7,28 (s, 1H), 7,43 (d, 1H), 7,89 (d, 1H).

### EXEMPLE 62 :

### Acide 4-(3-(2-hydroxy-éthoxy)-5,5,8,8-tétraméthyl-5,6,7,8-tétrahydronaphthalen-2-ylselanyl)-benzoïque

De manière analogue à l'exemple 2, par réaction de 322 mg (0,62 mmol) de 4-[3-(2-acétoxy-éthoxy)-5,5,8,8-tétraméthyl-5,6,7,8-tétrahydro-naphthalen-2-ylselanyl]-benzoate d'éthyle avec 250 mg (6,2 mmol) de soude dans un mélange THF/éthanol (3/3ml), on obtient 226 mg (81%) de poudre blanche. Tf :197°C.

### EXEMPLE 63 :

### 4-(3-(2-chloro-éthoxy)-5,5,8,8-tétraméthyl-5,6,7,8-tétrahydro-naphthalen-2-ylselanyl)-benzoate d'éthyle

De manière analogue à l'exemple 51, par réaction de 431 mg (1 mmol) de 4-(3-hydroxy-5,5,8,8-tétraméthyl-5,6,7,8-tétrahydro-naphthalen-2-ylselanyl-benzoate d'éthyle avec 222 mg (1,5 mmol) 1-bromo-2-chloroéthyle et de 278 mg de carbonate de potassium dans la MEC (20ml), on obtient 200 mg (40%) d'une huile jaune.

### EXEMPLE 64 :

### 4-[3-(2-iodo-éthoxy)-5,5,8,8-tétraméthyl-5,6,7,8-tétrahydro-naphthalen-2-ylselanyl]-benzoate d'éthyle

Un mélange de 200 mg (0,4 mmol) de 4-[3-(2-chloroéthoxy)-5,5,8,8-tétraméthyl-5,6,7,8-tétrahydro-naphthalen-2-ylselanyl]-benzoate d'éthyle avec 607 mg (4 mmol) d'iodure de sodium dans la MEC (4ml), est chauffé au reflux 12h. Le milieu réactionnel est traité à l'eau et à l'éther éthylique. La phase organique est lavée à l'eau, séchée sur sulfate de magnésium et concentrée à l'évaporateur rotatif. L'huile obtenue est remise en réaction dans les mêmes conditions. On obtient 159 mg (68%) d'un solide jaune.Pf=87°C.

### EXEMPLE 65 :

### Acide 6-(3-Adamantan-1-yl-4-methoxy-phenylselanyl)-nicotinique

Le produit est obtenu de manière analogue à l'exemple 7, à partir de 3-Adamantan-1-yl-4-méthoxy-phenyl disélénide et de 6-iodonicotinate d'éthyle.
RMN ¹H/THF D8: 1,79 (s, 6H), 2,04 (s, 3H), 2,13 (s, 6H), 3,89 (s, 3H), 6,93 (d, 1H), 7,02 (d, 1H), 7,52 à 7,55 (m, 2H), 7,91 (dd, 1H), 8,9 (d, 1H).

### EXEMPLE 66 :

### [6-(3,5,5,8,8-Pentamethyl-5,6,7,8-tetrahydro-naphthalen-2-ylselanyl)-pyridin-3-yl]-methanol

Dans un ballon et sous courant d'azote, on introduit 3 g (7 mmoles) de 6-(3,5,5,8,8-Pentamethyl-5,6,7,8-tetrahydro-naphthalen-2-ylselanyl)-nicotinate d'éthyle 800 mg (20 mmoles) d'hydrure double de lithium et d'aluminium et 90 ml de THF. On chauffe à reflux pendant deux heures, refroidit le milieu réactionnel, hydrolyse l'exces d'hydrure et filtre le sel. Après évaporation du filtrat le résidu obtenu est recristallisé dans l'heptane. On recueille 1,36 g (50%) de [6-(3,5,5,8,8-Pentamethyl-5,6,7,8-tetrahydro-naphthalen-2-ylselanyl)-pyridin-3-yl]-methanol de point de fusion 110-1°C.

### EXEMPLE 67 :

### N-Ethyl-6-(3,5,5,8,8-pentamethyl-5,6,7,8-tetrahydro-naphthalen-2-ylselanyl)-nicotinamide

(a) chlorure de 6-(3,5,5,8,8-pentaméthyl-5,6,7,8-tetrahydro-naphtalen-2-ylselanyl)-nicotinoyle
   Dans un ballon, on introduit 2 g (5 mmoles) d'acide 6-(3,5,5,8,8-pentaméthyl-5,6,7,8-tetrahydro-naphtalen-2-ylselanyl)-nicotinique 20 ml de toluène 100 µl de DMF et 450 µl de chlorure de thionyle. On chauffe à reflux pendant une heure, évapore le milieu réactionnel. On recueille 100% de chlorure d'acide attendu qui sera utilisé tel quel dans la suite de la synthèse.
(b) N-Ethyl-6-(3,5,5,8,8-pentamethyl-5,6,7,8-tetrahydro-naphthalen-2-ylselanyl)-nicotinamide
   Par réaction de 2,1 g (5 mmoles) du chlorure d'acide précédent avec 1 ml d'éthylamine (70% dans l'eau) dans 20 ml de THF, on obtient 2,02 g (95%) de l'amide attendu de point de fusion 218-20°C.

### EXEMPLE 68 :

### Morpholin-4-yl-[6-(3,5,5,8,8-pentamethyl-5,6,7,8-tetrahydro-naphthalen-2-ylselanyl)-pyridin-3-yl]-methanone

Par réaction de 2,1 g (5 mmoles) du chlorure d'acide précédent avec 1 ml de morpholine dans 20 ml de THF, on obtient 2,17 g (93%) de l'amide attendu de point de fusion 147-8°C.

### EXEMPLE 69 :

### N-(4-Hydroxy-phenyl)-6-(3,5,5,8,8-pentamethyl-5,6,7,8-tetrahydronaphthalen-2-ylselanyl)-nicotinamide

Par réaction de 2,1 g (5 mmoles) du chlorure d'acide précédent avec 540 mg (5 mmoles) de 4-aminophenol dans 40 ml de THF en présence de 830 µl de triéthylamine, on obtient 2,35 g (96%) de l'amide attendu de point de fusion 223-25°C.

### EXEMPLE 70 :

### 6-(3,5,5,8,8-Pentamethyl-5,6,7,8-tetrahydro-naphthalen-2-ylselanyl)-pyridine-3-carbaldehyde

Par réaction de 890 mg (2,3 mmoles) de [6-(3,5,5,8,8-Pentamethyl-5,6,7,8-tetrahydro-naphthalen-2-ylselanyl)-pyridin-3-yl]-methanol avec 1,12 g (3 mmoles) de pyridinium di chromate dans 90 ml de dichlorométhane, on obtient après filtration sur silice 600 mg (68%) de 6-(3,5,5,8,8-Pentamethyl-5,6,7,8-tetrahydro-naphthalen-2-ylselanyl)-pyridine-3-carbaldehyde de point de fusion 150-2°C.

### B. EXEMPLES DE FORMULATION

### 1) VOIE ORALE

(a) On prépare la composition suivante sous la forme d'un comprimé de 0,8 g

| | |
|---|---|
| Composé de l'exemple 3 | 0,005 g |
| Amidon prégélatinisé | 0,265 g |
| Cellulose microcristalline | 0,300 g |
| Lactose | 0,200 g |
| Stéarate de magnésium | 0,030 g |

Pour le traitement de l'acné, on administrera à un individu adulte 1 à 3 comprimés par jour pendant 3 à 6 mois selon la gravité du cas traité.
(b) On prépare une suspension buvable, destinée à être conditionnée en ampoules de 5 ml

| | |
|---|---|
| Composé de l'exemple 12 | 0,050 g |
| Glycérine | 0,500 g |
| Sorbitol à 70 % | 0,500 g |
| Saccharinate de sodium | 0,010 g |
| Parahydroxybenzoate de méthyle | 0,040 g |
| Arôme | q.s. |
| Eau purifiée | q.s.p.5 ml |

Pour le traitement de l'acné, on administrera à un individu adulte 1 ampoule par jour pendant 3 mois selon la gravité du cas traité.
(c) On prépare la formulation suivante destinée à être conditionnée en gélules :

| | |
|---|---|
| Composé de l'exemple 5 | 0,025 g |
| Amidon de maïs | 0,060 g |
| Lactose q.s.p. | 0,300 g |

Les gélules utilisées sont constituées de gélatine, d'oxyde de titane et d'un conservateur.

Dans le traitement du psoriasis, on administrera à un individu adulte, 1 gélule par jour pendant 30 jours.

### 2) VOIE TOPIQUE

(a) On prépare la crème Eau-dans-Huile non ionique suivante :

| | |
|---|---|
| Composé de l'exemple 23 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p. | 100,000 g |

Cette crème sera appliquée sur une peau psoriatique 1 à 2 fois par jour pendant 30 jours.
(b) On prépare un gel en réalisant la formulation suivante :

| | |
|---|---|
| Composé de l'exemple 39 | 0,050 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p. | 100,000 g |

Ce gel sera appliqué sur une peau atteinte de dermatose ou une peau acnéique 1 à 3 fois par jour pendant 6 à 12 semaines selon la gravité du cas traité.
(c) On prépare une lotion antiséborrhéique en procédant au mélange des ingrédients suivants :

| | |
|---|---|
| Composé de l'exemple 6 | 0,030 g |
| Propylène glycol | 5,000 g |
| Butylhydroxytoluène | 0,100 g |
| Ethanol (à 95°) q.s.p. | 100,000 g |

Cette lotion sera appliquée deux fois par jour sur un cuir chevelu séborrhéique et on constate une amélioration significative dans un délai compris entre 2 et 6 semaines.
(d) On prépare une composition cosmétique contre les effets néfastes du soleil en procédant au mélange des ingrédients suivants :

| | |
|---|---|
| Composé de l'exemple 59 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p. | 100,000 g |

Cette composition sera appliquée quotidiennement, elle permet de lutter contre le vieillissement photo-induit.
(e) On prépare la crème Huile dans l'Eau non ionique suivante :

| | |
|---|---|
| Composé de l'exemple 16 | 0,500 g |
| Vitamine D3 | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p. | 100.000 g |

Cette crème sera appliquée sur une peau psoriatique 1 à 2 fois par jour pendant 30 Jours.
(f) On prépare un gel topique en procédant au mélange des ingrédients suivants :

| | |
|---|---|
| Composé de l'exemple 4 | 0,050 g |
| Ethanol | 43,000 g |
| α -tocophérol | 0,050 g |
| Polymère carboxyvinylique vendu sous la dénomination "Carbopol 941" par la société "Goodrich" | 0,500 g |
| Triéthanolamine en solution aqueuse à 20 % en poids | 3,800 g |
| Eau | 9,300 g |
| Propylène glycol qsp | 100,000 g |

Ce gel sera appliqué dans le traitement de l'acné 1 à 3 fois par jour pendant 6 à 12 semaines selon la gravité du cas traité.
(g) On prépare une lotion capillaire anti-chute et pour la repousse des cheveux en procédant au mélange des ingrédients suivants :

| | |
|---|---|
| Composé de l'exemple 31 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100.00 g |

On appliquera cette lotion 2 fois par jour pendant 3 mois sur un cuir chevelu ayant subi une chute de cheveu importante.
(h) On prépare une crème anti-acnéique en procédant au mélange des ingrédients suivants :

| | |
|---|---|
| Composé de l'exemple 7 | 0,050 g |
| Acide rétinoïque. | 0.010 g |
| Mélange de stéarates de glycérol et de polyéthylène glycol (75 moles) vendu sous le nom de "Gelot 64" par la société "GATTEFOSSE" | 15,000 g |
| Huile de noyau polyoxyéthylénée à 6 moles d'oxyde d'éthylène vendue sous le nom de "Labrafil M2130 CS" par la société "GATTEFOSSE" | 8.000 g |
| Perhydrosqualène | 10,000 g |
| Conservateurs | qs |
| Polyéthylèneglycol (masse moléculaire = 400) | 8.000 g |
| Sel disodique de l'acide éthylène-diamine tétracétique | 0,050 g |
| Eau purifiée qsp | 100,000g |

Cette crème sera appliquée sur une peau atteinte de dermatose ou une peau acnéique 1 à 3 fois par jour pendant 6 à 12 semaines.
(i) On prépare une crème huile dans l'eau en réalisant la formulation suivante :

| | |
|---|---|
| Composé de l'exemple 43 | 0,020 g |
| 17-valérate de bétamethasone | 0.050 g |
| S-carboxyméthyl cystéine | 3, 000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p. | 100,000g |

Cette crème sera appliquée 2 fois par jour sur une peau atteinte de dermatose pendant 30 jours.
(j) On prépare la crème de type huile dans l'eau suivante :

| | |
|---|---|
| Acide lactique | 5,000 g |
| Composé de l'exemple 1 | 0,020g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" . | 4,200 g |
| Propylène glycol | 10,000 g |
| Butylhydroxyanisole | 0,010 g |
| Butylhydroxytoluène | 0.020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000g |
| Eau | q.s.p |
| | 100,000g |

Cette crème sera appliquée 1 fois par jour, elle aide à lutter contre le vieillissement qu'il soit photo-induit ou chronologique.

## Revendications

1. Composés, **caractérisés par le fait qu'**ils répondent à la formule générale (I) suivante: 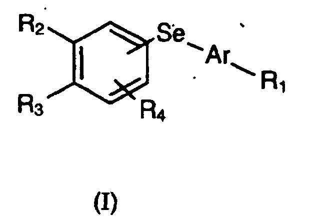 dans laquelle:
- R₁ représente :
(i) le radical -CH₃
(ii) le radical -CH₂-O-R₅
(iii) le radical -COR₆
R₅ et R₆ ayant les significations données ci-après,
- Ar représente un radical choisi parmi les radicaux de formules (a)-(e) suivantes 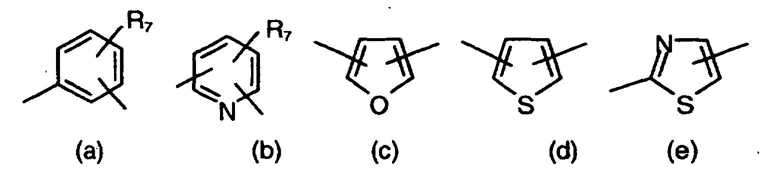 R₇ ayant la signification donnée ci-après,
- R₂ et R₃ identiques ou différents indépendamment représentent un radical choisi parmi :
(i) un atome d'hydrogène,
(ii) un radical choisi parmi les radicaux tertiobutyle, 1-méthylcyclohexyl, ou 1-Adamantyl,
(iii) un radical -OR₈ , R₈ ayant la signification donnée ci-après,
(iv) un radical polyéther de 1 à 6 atomes de carbone et de 1 à 3 atomes d'oxygène ou de soufre,
étant entendu qu'au moins un des radicaux R₂ ou R₃ représentent un radical (ii),
- R₂ et R₃ pris ensemble peuvent former avec le cycle aromatique adjacent un cycle saturé à 5 ou 6 chaînons éventuellement substitué par des groupes méthyles et / ou éventuellement interrompu par un atome d'oxygène ou de soufre,
- R₄ représente un atome d'hydrogène, un atome d'halogène, un radical alkyle de 1 à 6 atomes de carbone, un radical OR₉, un radical polyéther de 1 à 6 atomes de carbone et de 1 à 3 atomes d'oxygène ou de soufre, ou un radical COR₁₀,
R₉ et R₁₀ ayant les significations données ci-après,
- R₅ représente un atome d'hydrogène, un radical alkyle de 1 à 6 atomes de carbone, ou un radical COR₁₁
R₁₁ ayant la signification donnée ci-après,
- R₆ représente un radical choisi parmi:
(i) un atome d'hydrogène,
(ii) un radical alkyle de 1 à 6 atomes de carbone,
(iii) un radical OR₁₂
R₁₂ ayant la signification donnée ci-après,
(iv) un radical de formule 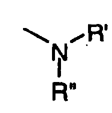 R' et R" ayant les significations données ci-après,
- R₇ représente un atome d'hydrogène, un atome d'halogène, un radical alkyle de 1 à 6 atomes de carbone, un radical nitro, un radical OR₁₃, un radical polyéther de 1 à 6 atomes de carbone et de 1 à 3 atomes d'oxygène ou de soufre ou un radical 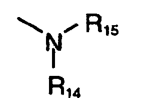 R₁₃, R₁₄, R₁₅ ayant les significations données ci-après,
- R₈ représente un atome d'hydrogène, un radical alkyle de 1 à 6 atomes de carbone, un radical aryle éventuellement substitué, un radical aralkyle éventuellement substitué, un radical monohydroxyalkyle de 1 à 6 atomes de carbone ou polyhydroxyalkyle de 2 à 6 atomes de carbone et de 2 à 5 groupes hydroxyles, ou un radical acyle de 1 à 4 atomes de carbone,
- R₉ représente un atome d'hydrogène, un radical alkyle de 1 à 6 atomes de carbone, un radical aryle éventuellement substitué, un radical aralkyle éventuellement substitué, un radical monohydroxyalkyle de 1 à 6 atomes de carbone ou polyhydroxyalkyle de 2 à 6 atomes de carbone et de 2 à 5 groupes hydroxyles, un radical acyle de 1 à 4 atomes de carbone, un radical -(CH₂)n-COOR₁₆, ou un radical -(CH₂)n-X,
n, R₁₆ et X ayant les significations données ci-après,
- R₁₀ et R₁₁, identiques ou différents, représentent un radical alkyle de 1 à 6 atomes de carbone,
- R₁₂ représente un atome d'hydrogène, un radical alkyle de 1 à 6 atomes de carbone, un radical aryle ou aralkyle éventuellement substitué, un radical monohydroxyalkyle de 1 à 6 atomes de carbone ou un radical polyhydroxyalkyle de 2 à 6 atomes de carbone et de 2 à 5 groupes hydroxyles,
- R' et R", identiques ou différents, représentent un atome d'hydrogène, un radical alkyle de 1 à 6 atomes de carbone, un radical aryle éventuellement substitué, ou un reste d'aminoacide,
ou encore R' et R" pris ensemble peuvent former avec l'atome d'azote un hétérocycle,
- R₁₃ représente un atome d'hydrogène, ou un radical alkyle de 1 à 6 atomes de carbone,
- R₁₄ et R₁₅, identiques ou différents, représentent un atome d'hydrogène, ou un radical alkyle de 1 à 6 atomes de carbone,
- R₁₆ représente un atome d'hydrogène, ou un radical alkyle de 1 à 6 atomes de carbone,
- n représente un entier compris entre 1 et 12 inclus,
- X représente un atome d'halogène,
et les isomères optiques et géométriques desdits composés de formule (I) ainsi que leurs sels.

2. Composés selon la revendication 1, **caractérisés par le fait qu'**ils se présentent sous forme de sels d'un métal alcalin ou alcalino-terreux, de zinc, d'une amine organique ou d'un acide minéral ou organique.

3. Composés selon l'une des revendications 1 ou 2, **caractérisés par le fait que** les radicaux alkyles de 1 à 6 atomes de carbone sont choisis parmi les radicaux méthyle, éthyle, isopropyle, butyle, ou tertiobutyle.

4. Composés selon l'une des revendications précédentes, **caractérisés en ce que** les radicaux monohydroxyalkyles de 1 à 6 atomes de carbone correspondent à des radicaux présentant 2 ou 3 atomes de carbone, notamment un radical 2-hydroxyéthyle, 2-hydroxypropyle ou 3-hydroxypropyle, le radical monohydroxyalkyle pouvant être protégé sous forme d'acétyle ou de tert-butyldiméthylsilyle.

5. Composés selon l'une des revendications précédentes, **caractérisés en ce que** les radicaux polyhydroxyalkyles de 2 à 6 atomes de carbone et de 2 à 5 groupes hydroxyles sont choisis parmi les radicaux 2,3-dihydroxypropyle, 2,3,4-trihydroxybutyle, 2,3,4,5-tétrahydroxypentyle ou le reste du pentaérythritol, les groupes hydroxyles pouvant être protégés sous forme d'acétyles ou de tert-butyldiméthylsilyles.

6. Composés selon l'une des revendications précédentes, **caractérisés en ce que** les radicaux aryles correspondent à un radical phényle, éventuellement substitué par au moins un halogène, un hydroxyle, ou une fonction nitro.

7. Composés selon l'une des revendications précédentes, **caractérisés en ce que** les radicaux aralkyles sont choisis parmi le radical benzyle ou phénéthyle éventuellement substitués par au moins un halogène, un hydroxyle, une fonction nitro.

8. Composés selon l'une des revendications précédentes, **caractérisés en ce que** les radicaux acyles de 1 à 4 atomes de carbone sont choisis parmi le radical acétyle ou le radical propionyle.

9. Composés selon l'une quelconque des revendications précédentes, **caractérisés en ce que** les radicaux polyéthers de 1 à 6 atomes de carbone et de 1 à 3 atomes d'oxygène ou de soufre sont choisis parmi les radicaux méthoxyméthyl éther, méthoxyéthoxyméthyl éther ou méthylthiométhyl éther.

10. Composés selon l'une quelconque des revendications précédentes, **caractérisés en ce que** les restes d'aminoacide sont choisis dans le groupe constitué par les restes dérivant de la lysine, de la glycine ou de l'acide aspartique.

11. Composés selon l'une quelconque des revendications précédentes, **caractérisés en ce que** les radicaux hétérocycliques sont choisis dans le groupe constitué par les radicaux pipéridino, morpholino, pyrrolidino ou pipérazino, éventuellement substitués en position 4 par un radical alkyle en C₁-C₆ ou par un mono- ou polyhydroxyalkyle.

12. Composés selon la revendication 1, **caractérisés par le fait qu'**ils sont pris, seuls ou en mélanges, dans le groupe constitué par :
4-(3,5,5,8,8-Pentamethyl-5,6,7,8-tetrahydro-naphthalen-2-ylselanyl)-benzoate d'ethyle,
acide 4-(3,5,5,8,8-Pentamethyl-5,6,7,8-tetrahydro-naphthalen-2-ylselanyl)-benzoique,
6-(3,5,5,8,8-Pentamethyl-5,6,7,8-tetrahydro-naphthalen-2-ylselanyl)-nicotinate d'ethyle,
acide 6-(3,5,5,8,8-pentaméthyl-5,6,7,8-tetrahydro-naphtalen-2-ylselanyl)-nicotinique,
6-(5,5,8,8-Tetramethyl-3-propoxy-5,6,7,8-tetrahydro-naphthalen-2-ylselanyl)-nicotinate d'ethyle,
acide 6-(5,5,8,8-Tetramethyl-3-propoxy-5,6,7,8-tetrahydro-naphthalen-2-ylselanyl-nicotinique,
acide 3-(4-tert-Butyl-phenylselanyl)-benzoique,
acide 6-(4-tert-Butyl-phenylselanyl)-nicotinique,
acide 4-(4-tert-Butyl-phenylselanyl)-benzoique,
acide 4-(4,4-Dimethyl-thiochroman-8-ylselanyl)-benzoique,
acide 3-(4,4-Dimethyl-thiochroman-8-ylselanyl)-benzoique,
acide 6-(4,4-Dimethyl-thiochroman-8-ylselanyl)-nicotinique,
acide 4-(5,5,8,8-Tetramethyl-5,6,7,8-tetrahydro-naphthalen-2-ylselanyl)-benzoique,
acide 3-(5,5,8,8-Tetramethyl-5,6,7,8-tetrahydro-naphthalen-2-ylselanyl)-benzoique,
acide 6-(5,5,8,8-Tetramethyl-5,6,7,8-tetrahydro-naphthalen-2-ylselanyl)-nicotinique,
acide 4-[5-Adamantan-1-yl-4-(2-methoxy-ethoxymethoxy)-2-methylphenylselanyl] -benzoique,
acide 3-[5-Adamantan-1-yl-4-(2-methoxy-ethoxymethoxy)-2-methylphenylselanyl] -benzoique,
acide 6-(4-Méthoxyéthoxyméthoxy-5,5,8,8-tétraméthyl-5,6,7,8-tétrahydronaphthalen-2-ylselanyl)-nicotinique,
acide 3-(4-Méthoxyéthoxyméthoxy-5,5,8,8-tétramethyl-5,6,7,8-tétrahydronaphthalen-2-ylselanyl)-benzoïque,
acide 4-(4-Méthoxyéthoxyméthoxy-5,5,8,8-tétraméthyl-5,6,7,8-tétrahydronaphthalen-2-ylselanyl)-3-méthoxy-benzoïque,
acide 3-(4-Méthoxyéthoxyméthoxy-5,5,8,8-tétraméthyl-5,6,7,8-tétrahydronaphthalen-2-ylselanyl)-4-méthoxy-benzoïque,
acide 6-(4-Méthoxyméthoxy-5,5,8,8-tétraméthyl-5,6,7,8-tétrahydro-naphthalen-2-ylselanyl)-nicotinique,
acide 6-(3-Méthoxyéthoxyméthoxy-5,5,8,8-tétraméthyl-5,6,7,8-tétrahydronaphthalen-2-ylselanyl)-nicotinique,
acide 2-(3-Méthoxyéthoxyméthoxy-5,5,8,8-tétraméthyl-5,6,7,8-tétrahydronaphthalen-2-ylselanyl)-nicotinique,
acide 4-(3-Méthoxyéthoxyméthoxy-5,5,8,8-tétraméthyl-5,6,7,8-tétrahydronaphthalen-2-ylselanyl)-benzoïque,
acide 3-(3-Méthoxyéthoxyméthoxy-5,5,8,8-tétraméthyl-5,6,7,8-tétrahydronaphthalen-2-ylselanyl)-benzoïque,
acide 6-(3,5-Di-tert-butyl-2-méthoxyméthoxy-phénylselanyl)-nicotinique,
acide 2-(3,5-Di-tert-butyl-2-méthoxyméthoxy-phénylselanyl)-nicotinique,
acide 4-(3,5-Di-tert-butyl-2-méthoxyméthoxy-phénylselanyl)-benzoïque,
acide 3-(3,5-Di-tert-butyl-2-méthoxyméthoxy-phénylselanyl)-benzoïque,
acide 6-[4-adamantan-1-yl-3-benzyloxy-phenylsélenalyl]-nicotinique,
acide 6-(3,5-Di-tert-butyl-2-benzyloxy-phénylselanyl)-nicotinique,
acide 3-méthoxy-4-(4-benzyloxy-5,6,7,8-tétrahydro-5,5,8,8-tétraméthylnaphtalen-2-ylsélanyl)-benzoïque,
acide 4-(4-benzyloxy-5,6,7,8-tétrahydro-5,5,8,8-tétraméthylnaphtalen-2-ylsélanyl)-benzoïque,
acide 6-(4-benzyloxy-5,6,7,8-tétrahydro-5,5,8,8-tétraméthylnaphtalen-2-ylsélanyl)-nicotinique,
acide 3-méthoxy-4-(3-benzyloxy-5,6,7,8-tétrahydro-5,5,8,8-tétraméthylnaphtalen-2-ylsélanyl)-benzoïque,
acide 6-(3-benzyloxy-5,6,7,8-tétrahydro-5,5,8,8-tétraméthylnaphtalen-2-ylsélanyl)-nicotinique,
acide 4-(3-hexyloxy-5,6,7,8-tétrahydro-5,5,8,8-tétraméthylnaphtaten-2-ylsélanyl)3-méthoxy-benzoïque,
acide 6-(3-hexyloxy-5,6,7,8-tétrahydro-5,5,8,8-tétraméthylnaphtalen-2-ylsélanyl)-nicotinique,
acide 4-(5-adamantan-1-yl-4-benzyloxy-2-méthyl-phenylselenatyl)-benzoïque,
acide 6-[3-(5-Hydroxy-pentyloxy)-5,5,8,8-tetramethyl-5,6,7,8-tetrahydronaphthalen-2-ylselanyl]- nicotinique,
4-(5,5,8,8-Tetramethyl-5,6,7,8-tetrahydro-naphthalen-2-ylselanyl)-benzoate d'éthyle,
4-(3-Méthoxyéthoxyméthoxy-5,5,8,8-tétraméthyl-5,6,7,8-tétrahydro-naphthalen-2-ylselanyl)-benzoate d'éthyle,
4-(3-Hydroxy-5,5,8,8-tetramethyl-5,6,7,8-tetrahydro-naphthalen-2-ylselanyl)-benzoate d'éthyle,
acide 4-(3-Hydroxy-5,5,8,8-tetramethyl-5,6,7,8-tetrahydro-naphthalen-2-ylselanyl) -benzoïque,
6-(3-Méthoxyéthoxyméthoxy-5,5,8,8-tétraméthyl-5,6,7,8-tétrahydro-naphthalen-2-ylselanyl)-nicotinate d'éthyle,
6-(3-Hydroxy-5,5,8,8-tetramethyl-5,6,7,8-tetrahydro-naphthalen-2-ylselanyl)-nicotinate d'éthyle,
acide 6-(3-Hydroxy-5,5,8,8-tetramethyl-5,6,7,8-tetrahydro-naphthalen-2-ylselanyl) -nicotinique,
6-[3-(3-Ethoxycarbonyl-propoxy)-5,5,8,8-tetramethyl-5,6,7,8-tetrahydronaphthalen-2- ylselanyl]-nicotinate d'éthyle,
acide 6-[3-(3-Carboxy-propoxy)-5,5,8,8-tetramethyl-5,6,7,8-tetrahydronaphthalen -2-ylselanyl]- nicotinique,
4-[3-(3-Ethoxycarbonyl-propoxy)-5,5,8,8-tetramethyl-5,6,7,8-tetrahydronaphthalen-2- ylselanyl]-benzoate d'éthyle,
acide 4-[3-(3-Carboxy-propoxy)-5,5,8,8-tetramethyl-5,6,7,8-tetrahydronaphthalen -2-ylselanyl]- benzoique,
4-[3-(7-Methoxycarbonyl-heptyloxy)-5,5,8,8-tetramethyl-5,6,7,8-tetrahydronaphthalen-2- ylselanyl]-benzoate d'éthyle,
acide 4-[3-(7-Carboxy-heptyloxy)-5,5,8,8-tetramethyl-5,6,7,8-tetrahydronaphthalen-2-ylselanyl]- benzoique,
6-[3-(7-Methoxycarbonyl-heptyloxy)-5,5,8,8-tetramethyl-5,6,7,8-tetrahydronaphthalen-2- ylselanyl]-nicotinate d'éthyle,
acide 6-[3-(7-Carboxy-heptyloxy)-5,5,8,8-tetramethyl-5,6,7,8-tetrahydronaphthalen-2-ylselanyl]- nicotinique,
6-[3-(2-Acetoxy-ethoxy)-5,5,8,8-tetramethyl-5,6,7,8-tetrahydro-naphthalen-2-ylselanyl]- nicotinate d'éthyle,
acide 6-[3-(2-Hydroxy-ethoxy)-5,5,8,8-tetramethyl-5,6,7,8-tetrahydronaphthalen-2-ylselanyl]- nicotinique,
4-[3-(2-Acetoxy-ethoxy)-5,5,8,8-tetramethyl-5,6,7,8-tetrahydro-naphthalen-2-ylselanyl]-benzoate d'éthyle,
acide 4-[3-(2-Hydroxy-ethoxy)-5,5,8,8-tetramethyl-5,6,7,8-tetrahydronaphthalen-2-ylselanyl]-benzoique,
acide 6-(3-Adamantan-1-yl-4-methoxy-phenylselanyl)-nicotinique,
[6-(3,5,5,8,8-Pentamethyl-5,6,7,8-tetrahydro-naphthalen-2-ytselanyl)-pyridin-3-yl]-methanol,
N-Ethyl-6-(3,5,5,8,8-pentamethyl-5,6,7,8-tetrahydro-naphthalen-2-ylselanyl)-nicotinamide,
Morpholin-4-yl-[6-(3,5,5,8,8-pentamethyl-5,6,7,8-tetrahydro-naphthalen-2-ylselanyl)-pyridin-3-yl]-methanone,
N-(4-Hydroxy-phenyl)-6-(3,5,5,8,8-pentamethyl-5,6,7,8-tetrahydro-naphthalen-2-ylselanyl)-nicotinamide,
6-(3,5,5,8,8-Pentamethyl-5,6,7,8-tetrahydro-naphthalen-2-ylselanyl)-pyridine-3-carbaldehyde,

13. Composés selon la revendication 1, **caractérisés par le fait qu'**ils présentent l'une au moins l'une des caractéristiques suivantes :
- R₁ représente un radical COR₆
- Ar représente un radical de formule (a) ou (b)
- R₂ ou R₃ représentent un radical adamantyle ou R₂ et R₃ pris ensemble forment avec le cycle aromatique adjacent un cycle saturé à 5 ou 6 chaînons éventuellement substitué par des groupes méthyle et / ou éventuellement interrompu par un atome d'oxygène ou de soufre.

14. Composés selon la revendication 13, **caractérisés par le fait qu'**ils présentent toutes les caractéristiques suivantes :
- R₁ représente un radical COR₆
- Ar représente un radical de formule (a) ou (b)
- R₂ ou R₃ représentent un radical adamantyle ou R₂ et R₃ pris ensemble forment avec le cycle aromatique adjacent un cycle saturé à 5 ou 6 chaînons éventuellement substitué par des groupes méthyle et / ou éventuellement interrompu par un atome d'oxygène ou de soufre.

15. Composés selon l'une quelconque des revendications précédentes pour une utilisation comme médicament.

16. Composés selon la revendication 15 pour une utilisation comme médicament destiné au traitement des affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle; pour traiter d'autres types de troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal); pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale, les composés peuvent également être utilisés dans certaines affections inflammatoires ne présentant pas de trouble de la kératinisation; pour traiter toutes les proliférations dermiques ou épidermiques qu'elles soient bénignes ou malignes, qu'elles soient ou non d'origine virale telles que les verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires; pour traiter d'autres désordres dermatologiques tels que les dermatoses bulleuses et les maladies du collagène; pour traiter certains troubles ophtalmologiques, notamment les cornéopathies; pour réparer ou lutter contre le vieillissement de la peau, qu'il soit photoinduit ou chronologique ou pour réduire les pigmentations et les kératoses actiniques, ou toutes pathologies associées au vieillissement chronologique ou actinique; pour prévenir ou guérir les stigmates de l'atrophie épidermique et/ou dermique induite par les corticostéroïdes locaux ou systémiques, ou tout autre forme d'atrophie cutanée, pour prévenir ou traiter les troubles de la cicatrisation ou pour prévenir ou réparer les vergetures; pour favoriser la cicatrisation, pour lutter contre les troubles de la fonction sébacée tels que l'hyperséborrhée de l'acné ou la séborrhée simple; pour le traitement ou la prévention des états cancéreux ou précancéreux, plus particulièrement les leucémies promyétocytaires; pour le traitement d'affections inflammatoires telles que l'arthrite, pour le traitement de toute affection d'origine virale au niveau cutané ou général; pour la prévention ou le traitement de l'alopécie; pour le traitement d'affections dermatologiques à composante immunitaire; pour le traitement d'affections du système cardiovasculaire telles que l'artériosclérose, l'hypertension, le diabète non-insulino dépendant ainsi que l'obésité, pour le traitement de désordres cutanés dus à une exposition aux rayonnements U.V..

17. Composition pharmaceutique, **caractérisée par le fait qu'**elle comprend, dans un support pharmaceutiquement acceptable, au moins l'un des composés tels que définis à l'une quelconque des revendications 1 à 14.

18. Composition selon la revendication 17, **caractérisée en ce que** la concentration en composé(s) selon l'une des revendications 1 à 14 est comprise entre 0,001 % et 5 % en poids par rapport à l'ensemble de la composition.

19. Composition cosmétique, **caractérisée par le fait qu'**elle comprend, dans un support cosmétiquement acceptable, au moins l'un des composés tels que définis à l'une quelconque des revendications 1 à 14.

20. Composition selon la revendication 19, **caractérisée en ce que** la concentration en composé(s) selon l'une des revendications 1 à 14 est comprise entre 0,001 % et 3 % en poids par rapport à l'ensemble de la composition.

21. Utilisation d'une composition cosmétique telle que définie à l'une des revendications 19 ou 20 pour l'hygiène corporelle ou capillaire.

## Patentansprüche

1. Verbindungen, **dadurch gekennzeichnet, dass** sie der folgenden allgemeinen Formel (I) entsprechen: 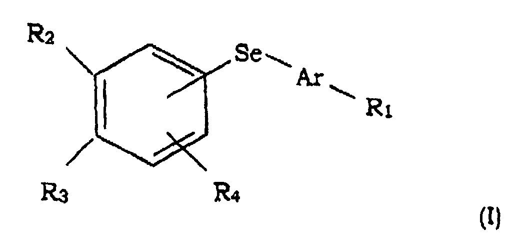 worin bedeuten:
- R₁:
(i) die Gruppe -CH₃
(ii) die Gruppe -CH₂O-R₅
(iii) die Gruppe -COR₆,
wobei R₅ und R6 die nachstehend angegebenen Bedeutungen aufweisen:
- Ar eine Gruppe, die unter den Gruppen der folgenden FormeIn (a) bis (e) ausgewählt sind, 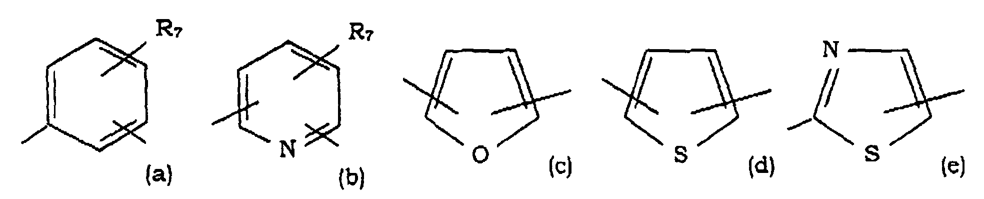 wobei R₇ die nachstehend angegebene Bedeutung aufweist,
- die Gruppen R₂ und R₃, die gleich oder verschieden sind, unabhängig voneinander eine Gruppe, die ausgewählt ist unter:
(i) einem Wasserstoffatom,
(ii) einer Gruppe, die unter den Gruppen t-Butyl, 1-Methylcyclohexyl oder 1-Adamantyl ausgewählt ist,
(iii) einer Gruppe -OR₈, wobei R₈ die nachstehend angegebene Bedeutung aufweist,
(iv) einer Polyethergruppe mit 1 bis 6 Kohlenstoffatomen und 1 bis 3 Sauerstoffatomen oder Schwefelatomen,
mit der Maßgabe, dass mindestens eine der Gruppen R₂ oder R₃ die Gruppe (ii) bedeutet,
- wobei R₂ und R₃ gemeinsam mit dem angrenzenden aromatischen Ring einen gesättigten 5- oder 6-gliedrigen Ring bilden können, der gegebenenfalls mit Methylgruppen substituiert und/ oder gegebenenfalls durch ein Sauerstoffatom oder Schwefelatom unterbrochen ist,
- R₄ ein Wasserstoffatom, ein Halogenatom, eine Alkylgruppe mit 1 bis 6 Kohlenstoffatomen, eine Gruppe OR₉, eine Polyethergruppe mit 1 bis 6 Kohlenstoffatomen und 1 bis 3 Sauerstoffatomen oder Schwefelatomen oder eine Gruppe COR₁₀, wobei R₉ und R₁₀ die nachstehend angegebenen Bedeutungen aufweisen
- Rs ein Wasserstoffatom, eine Alkylgruppe mit 1 bis 6 Kohlenstoffatomen oder eine Gruppe COR₁₁, wobei R₁₁ die nachstehend angegebenen Bedeutungen aufweist,
- R₆ eine Gruppe, die ausgewählt ist unter:
(i) einem Wasserstoffatom,
(ii) einer Alkylgruppe mit 1 bis 6 Kohlenstoffatomen,
(iii) einer Gruppe OR₁₂, wobei R₁₂ die nachstehend angegebene Bedeutung aufweist,
(iv) eine Gruppe der Formel 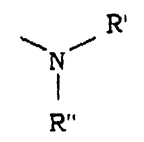 wobei R' und R" die nachstehend angegebenen Bedeutungen aufweisen,
- R₇ ein Wasserstoffatom, ein Halogenatom, eine Alkylgruppe mit 1 bis 6 Kohlenstoffatomen, eine Nitrogruppe, eine Gruppe OR₁₃, eine Polyethergruppe mit 1 bis 6 Kohlenstoffatomen und 1 bis 3 Sauerstoffatomen oder Schwefelatomen oder eine Gruppe 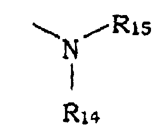 wobei R₁₃, R₁₄ und R₁₅ die nachstehend angegebenen Bedeutungen aufweisen,
- R₈ ein Wasserstoffatom, eine Alkylgruppe mit 1 bis 6 Kohlenstoffatomen, eine gegebenenfalls substituierte Arylgruppe, eine gegebenenfalls substituierte Aralkylgruppe, eine Monohydroxyalkylgruppe mit 1 bis 6 Kohlenstoffatomen, eine Polyhydroxyalkylgruppe mit 2 bis 6 Kohlenstoffatomen und 2 bis 5 Hydroxygruppen oder eine Acylgruppe mit 1 bis 4 Kohlenstoffatomen,
- R₉ ein Wasserstoffatom, eine Alkylgruppe mit 1 bis 6 Kohlenstoffatomen, eine gegebenenfalls substituierte Arylgruppe, eine gegebenenfalls substituierte Aralkylgruppe, eine Monohydroxyalkylgruppe mit 1 bis 6 Kohlenstoffatomen, eine Polyhydroxyalkylgruppe mit 2 bis 6 Kohlenstoffatomen und 2 bis 5 Hydroxygruppen, eine Acylgruppe mit 1 bis 4 Kohlenstoffatomen, eine Gruppe -(CH₂)ₙ-COOR₁₆ oder eine Gruppe -(CH₂)ₙ-X, wobei n, R₁₆ und X die nachstehend angegebenen Bedeutungen aufweisen,
- R₁₀ und R₁₁, die gleich oder verschieden sind, eine Alkylgruppe mit 1 bis 6 Kohlenstoffatomen,
- R₁₂ ein Wasserstoffatom, eine Alkylgruppe mit 1 bis 6 Kohlenstoffatomen, eine gegebenenfalls substituierte Aryl- oder Aralkylgruppe, eine Monohydroxyalkylgruppe mit 1 bis 6 Kohlenstoffatomen oder eine Polyhydroxyalkylgruppe mit 2 bis 6 Kohlenstoffatomen und 2 bis 5 Hydroxygruppen,
- R' und R", die identisch oder voneinander verschieden sind, ein Wasserstoffatom, eine Alkylgruppe mit 1 bis 6 Kohlenstoffatomen, eine gegebenenfalls substituierte Arylgruppe oder einen Aminosäurerest,
oder die Gruppen R' und R" bilden gemeinsam mit dem Stickstoffatom einen Heterocyclus,
- R₁₃ ein Wasserstoffatom oder eine Alkylgruppe mit 1 bis 6 Kohlenstoffatomen,
- R₁₄ und R₁₅, die gleich oder verschieden sind, ein Wasserstoffatom oder eine Alkylgruppe mit 1 bis 6 Kohlenstoffatomen,
- R₁₆ ein Wasserstoffatom oder eine Alkylgruppe mit 1 bis 6 Kohlenstoffatomen,
- n eine ganze Zahl im Bereich von 1 bis 12, wobei die Grenzen eingeschlossen sind,
- X ein Halogenatom,
und die optischen und geometrischen Isomere der Verbindungen der Formel (I) sowie deren Salze.

2. Verbindungen nach Anspruch 1, **dadurch gekennzeichnet, dass** sie in Form von Alkalimetallsalzen, Erdalkalimetallsalzen, Zinksalzen, Salzen von organischen Aminen oder Salzen von anorganischen oder organischen Säuren vorliegen.

3. Verbindungen nach einem der Ansprüche 1 oder 2, **dadurch gekennzeichnet, dass** die Alkylgruppen mit 1 bis 6 Kohlenstoffatomen unter Methyl, Ethyl, Isopropyl, Butyl oder *t*-Butyl ausgewählt sind.

4. Verbindungen nach einem der vorhergehenden Ansprüche, **dadurch gekennzeichnet, dass** die Monohydroxyalkylgruppen mit 1 bis 6 Kohlenstoffatomen Gruppen entsprechen, die 2 oder 3 Kohlenstoffatome enthalten, insbesondere 2-Hydroxyethyl, 2-Hydroxypropyl oder 3-Hydroxypropyl, wobei die Monohydroxyalkylgruppe in Form von Acetyl oder *t*-Butyldimethylsilyl geschützt sein kann.

5. Verbindungen nach einem der vorhergehenden Ansprüche, **dadurch gekennzeichnet, dass** die Polyhydroxyalkylgruppen mit 2 bis 6 Kohlenstoffatomen und 2 bis 5 Hydroxygruppen unter 2,3-Dihydroxypropyl, 2,3,4-Trihydroxybutyl, 2,3,4,5-Tetrahydroxypentyl oder Pentaerythrit ausgewählt sind, wobei die Hydroxygruppen in Form von Acetyl oder *t*-Butyldimethylsilyl geschützt sein können.

6. Verbindungen nach einem der vorhergehenden Ansprüche, **dadurch gekennzeichnet, dass** die Arylgruppen Phenylgruppen sind, die gegebenenfalls mit mindestens einem Halogen, einer Hydroxygruppe oder einer Nitrofunktion substituiert sind.

7. Verbindungen nach einem der vorhergehenden Ansprüche, **dadurch gekennzeichnet, dass** die Aralkylgruppen unter Benzyl oder Phenethyl ausgewählt sind, wobei diese gegebenenfalls mit mindestens einem Halogen, einer Hydroxygruppe oder einer Nitrofunktion substituiert sind.

8. Verbindungen nach einem der vorhergehenden Ansprüche, **dadurch gekennzeichnet, dass** die Acylgruppen mit 1 bis 4 Kohlenstoffatomen unter Acetyl oder Propionyl ausgewählt sind.

9. Verbindungen nach einem der vorhergehenden Ansprüche, **dadurch gekennzeichnet, dass** die Polyethergruppen mit 1 bis 6 Kohlenstoffatomen und 1 bis 3 Sauerstoffatomen oder Schwefelatomen unter den Gruppen Methoxymethylether, Methoxyethoxymethylether oder Methylthiomethylether ausgewählt sind.

10. Verbindungen nach einem der vorhergehenden Ansprüche, **dadurch gekennzeichnet, dass** die Aminosäurereste unter den Resten ausgewählt sind, die von Lysin, Glycin oder Asparaginsäure abgeleitet sind.

11. Verbindungen nach einem der vorhergehenden Ansprüche, **dadurch gekennzeichnet, dass** die heterocyclischen Gruppen unter Piperidino, Morpholino, Pyrrolidino oder Piperazino ausgewählt sind, wobei sie gegebenenfalls in 4-Stellung mit einer C₁₋₆-Alkylgruppe oder einer Mono- oder Polyhydroxyalkylgruppe substituiert sind.

12. Verbindungen nach Anspruch 1, **dadurch gekennzeichnet, dass** sie unter den folgenden Verbindungen oder deren Gemischen ausgewählt sind:
Ethyl-4-(3,5,5,8,8-pentamethyl-5,6,7,8-tetrahydro-naphthalin-2-ylselanyl)-benzoat,
4-(3,5,5,8,8-Pentamethyl-5,6,7,8-tetrahydro-naphthalin-2-ylselanyl)-benzoesäure,
Ethyl-6-(3,5,5,8,8-pentamethyl-5,6,7,8-tetrahydro-naphthalin-2-ylselanyl)-nicotinat,
6-(3,5,5,8,8-Pentamethyl-5,6,7,8-tetrahydro-naphthalin-2-ylselanyl)-nicotinsäure,
Ethyl-6-(5,5,8,8-tetramethyl-3-propoxy-5,6,7,8-tetrahydronaphthalin-2-ylselanyl)-nicontinat,
6-(5,5,8,8-Tetramethyl-3-propoxy-5,6,7,8-tetrahydro-naphthalin-2-ylselanyl)-nicotinsäure,
3-(4-*t*-Butyl-phenylselanyl)-benzoesäure,
6-(4-*t*-Butyl-phenylselanyl)-nicotinsäure,
4-(4-*t*-Butyl-phenylselanyl)-benzoesäure,
4-(4,4-Dimethyl-thiochroman-8-ylselanyl)-benzoesäure,
3-(4,4-Dimethyl-thiochroman-8-ylselanyl)-benzoesäure,
6-(4,4-Dimethyl- thiochroman-8-ylselanyl)-nicotinsäure,
4-(5,5,8,8-Tetramethyl-5,6,7,8-tetrahydro-naphthalin-2-ylselanyl)-benzoesäure,
3-(5,5,8,8-Tetramethyl-5,6,7,8-tetrahydro-naphthalin-2-ylselanyl)-benzoesäure,
6-(5,5,8,8-Tetramethyl-5,6,7,8-tetrahydro-naphthalin-2-ylselanyl)-nicotinsäure,
4-[5-Adamantan-1-yl-4-(2-methoxy-ethoxymethoxy)-2-methylphenylselanyl]-benzoesäure,
3-[5-Adamantan-1-yl-4- (2-methoxy-ethoxymethoxy)-2-methylphenylselanyl]-benzoesäure,
6-(4-Methoxyethoxymethoxy-5,5,8,8-tetramethyl-5,6,7,8-tetrahydro-naphthalin-2-ylselanyl)-nicotinsäure,
3-(4-Methoxyethoxymethoxy-5,5,8,8-tetramethyl-5,6,7,8-tetrahydro-naphthalin-2-ylselanyl)-benzoesäure,
4-(4-Methoxyethoxymethoxy-5,5,8,8-tetramethyl-5,6,7,8-tetrahydro-naphthalin-2-ylselanyl)-3-methoxy-benzoesäure,
3-(4-Methoxyethoxymethoxy-5,5,8,8-tetramethyl-5,6,7,8-tetrahydro-naphthalin-2-ylselanyl)-4-methoxy-benzoesäure,
6-(4-Methoxymethoxy-5,5,8,8-tetramethyl-5,6,7,8-tetrahydronaphthalin-2-ylselanyl)-nicotinsäure,
6-(3-Methoxyethoxymethoxy-5,5,8,8-tetramethyl-5,6,7,8-tetrahydro-naphthalin-2-ylselanyl)-nicotinisäure,
2-(3-Methoxyethoxymethoxy-5,5,8,8-tetramethyl-5,6,7,8-tetrahydro-naphthalin-2-ylselanyl)-nicotinsäure,
4-(3-Methoxyethoxymethoxy-5,5,8,8-tetramethyl-5,6,7,8-tetrahydro-naphthalin-2-ylselanyl)-benzoesäure,
3-(3-Methoxyethoxymethoxy-5,5,8,8-tetramethyl-5,6,7,8-tetrahydro-naphthalin-2-ylselanyl)-benzoesäure,
6-(3,5-Di-*t*-butyl-2-methoxymethoxy-phenylselanyl)-nicotinsäure,
2-(3,5-Di-*t*-butyl-2-methoxymethoxy-phenylselanyl)-nicotinsäure,
4-(3,5-Di-*t* butyl-2-methoxymethoxy-phenylselanyl)-benzoesäure,
3-(3,5-Di-*t*-butyl-2-methoxymethoxy-phenylselanyl)-benzoesäure,
6-[4-Adamantan-1-yl-3-benzyloxy-phenylselenalyl]-nicotinsäure,
6-(3, 5-Di-*t*-butyl-2-benzyloxy-phenylselanyl)-nicotinsäure,
3-Methoxy-4-(4-benzyloxy-5,6,7,8-tetrahydro-5,5,8,8-tetramethyl-naphthalin-2-ylselanyl)-benzoesäure,
4-(4-Benzyloxy-5,6,7,8-tetrahydro-5,5,8,8-tetramethylnaphthalin-2-ylselanyl)-benzoesäure,
6-(4-Benzyloxy-5,6,7,8-tetrahydro-5,5,8-tetramethylnaphthalin-2-ylselanyl)-nicotinsäure,
3-Methoxy-4-(3-benzyloxy-5,6,7,8-tetrahydro-5,5,8,8-tetramethyl-naphthalin-2-ylselanyl)-benzoesäure,
6-(3-Benzyloxy-5,6,7,8-tetrahydro-5,5,8,8-tetramethylnaphthalin-2-ylselanyl)-nicotinsäure,
4-(3-Hexyloxy-5,6,7,8-tetrahydro-5,5,8,8-tetramethylnaphthalin-2-ylselanyl)-3-methoxy-benzoesäure,
6-(3-Hexyloxy-5,6,7,8-tetrahydro-5,5,8,8-tetramethylnaphthalin-2-ylselanyl)-nicotinsäure,
4-(5-Aadamantan-1-yl-4-benzyloxy-2-methyl-phenylselenalyl)-benzoesäure,
6-[3-(5-Hydroxy-pentyloxy)-5,5,8,8-tetramethyl-5,6,7,8-tetrahydro-naphthalin-2-ylselanyl]-nicotinsäure,
Ethyl-4-(5,5,8,8-tetramethyl-5,6,7,8-tetrahydro-naphthalin-2-ylselanyl)-benzoat,
Ethyl-4-(3-methoxyethoxymethoxy-5,5,8,8-tetramethyl-5,6,7,8-tetrahydro-naphthalin-2-ylselanyl)-benzoat,
Ethyl-4-(3-Hydroxy-5,5,8,8-tetramethyl-5,6,7,8-tetrahydronaphthalin-2-ylselanyl)-benzoat,
4-(3-Hydroxy-5,5,8,8-tetramethyl-5,6,7,8-tetrahydro-naphthalin-2-ylselanyl)-benzoesäure,
Ethyl-6-(3-methoxyethoxymethoxy-5, 5,8,8-tetramethyl-5,6,7,8-tetrahydro-naphthalin-2-ylselanyl)-nicotinat,
Ethyl-6-(3-hydroxy-5,5,8,8-tetramethyl-5,6,7,8-tetrahydronaphthalin-2-ylselanyl)-nicotinat,
6-(3-Hydroxy-5,5,8,8-tetramethyl-5,6,7,8-tetryhydro-naphthalin-2-ylselanyl)-nicotinsäure,
Ethyl-6-[3-(3-ethoxycarbonyl-propoxy)-5,5,8,8-tetramethyl-5,6,7,8-tetrahydro-naphthalin-2-ylselanyl]-nicotinat,
6-[3-(3-Carboxy-propoxy)-5,5,8,8-tetramethyl-5,6,7,8-tetrahydronaphthalin-2-ylselanyl]-nicotinsäure,
Ethyl-4-[3-(3-ethoxycarbonyl-propoxy)-5,5,8,8-tetramethyl-5, 6,7,8-tetrahydro-naphthalin-2-ylselanyl]-benzoat,
4-[3-(3-Carboxy-propoxy)-5,5,8,8-tetramethyl-5,6,7,8-tetrahydronaphthalin-2-ylselanyl]-benzoesäure,
Ethyl-4-[3-(7-methoxycarbonyl-heptyloxy)-5,5,8,8-tetramethyl-5,6,7,8-tetrahydro-naphthalin-2- ylselanyl]-benzoat,
4-[3-(7-Carboxy-heptyloxy)-5,5,8,8-tetramethyl-5,6,7,8-tetrahydro-naphthalin-2-ylselanyl]-benzoesäure,
Ethyl-6-[3-(7-methoxycarbonyl-heptyloxy)-5,5,8,8-tetramethyl-5,6,7,8-tetrahydro-naphthalin-2-ylselanyl]-nicotinat,
6-[3-(7-Carboxy-heptyloxy)-5,5,8,8-tetramethyl-5,6,7,8-tetrahydro-naphthalin-2-ylselanyl]-nicotinsäure,
Ethyl-6-[3-(2-acetoxy-ethoxy)-5,5,8,8-tetramethyl-5,6,7,8-tetrahydro-naphthalin-2-ylselanyl]-nicotinat,
6-[3-(2-1-Hydroxy-ethoxy)-5,5,8,8-tetramethyl-5,6,7,8-tetrahydronaphthalin-2-ylselanyl]-nicotinsäure,
Ethyl-4-[3-(2-Acetoxy-ethoxy)-5,5,8,8-tetramethyl-5,6,7,8-tetrahydro-naphthalin-2-ylselanyl]-benzoat,
4-[3-(2-Hydroxy-ethoxy)-5,5,8,8-tetramethyl-5,6,7,8-tetrahydronaphthalin-2-ylselanyl]-benzoesäure,
6-(3-Adamantan-1-yl-4-methoxy-phenylselanyl)-nicotinsäure,
[6-(3,5,5,8,8-Pentamethyl-5,6,7,8-tetrahydro-naphthalin -2-ylselanyl)-pyridin-3-yl]-methanol,
N-Ethyl-6-(3,5,5,8,8-pentamethyl-5,6,7,8-tetrahydro-naphthalin-2-ylselanyl)-nicotinamid,
Morpholin-4-yl-[6-(3,5,5,8,8-pentamethyl-5,6,7,8-tetrahydronaphthalin-2-ylselanyl)-pyridin-3-yl]-methanon,
N -(4- Hydroxy-phenyl)-6-(3,5,5,8,8-pentamethyl-5,6,7,8-tetrahydro-naphthalin-2-ylselanyl)-nicotinamid,
6-(3,5,5,8,8-Pentamethyl-5,6,7,8-tetrahydro-naphthalin-2-ylselanyl)-pyridin-3-carbaldehyd.

13. Verbindungen nach Anspruch 1, **dadurch gekennzeichnet, dass** sie mindestens eine der folgenden Eigenschaften aufweisen:
- R₁ bedeutet eine Gruppe COR₆
- Ar bedeutet eine Gruppe der Formel (a) oder (b)
- R₂ und R₃ bedeuten Adamantyl oder R₂ und R₃ bilden gemeinsam mit dem angrenzenden aromatischen Ring einen gesättigten, 5- oder 6-gliedrigen Ring, der gegebenenfalls mit Methylgruppen substituiert und/oder gegebenenfalls durch ein Sauerstoffatom oder Schwefelatom unterbrochen ist.

14. Verbindung nach Anspruch 13, **dadurch gekennzeichnet, dass** sie alle folgenden Eigenschaften aufweisen:
- R₁ bedeutet eine Gruppe COR₆
- Ar bedeutet eine Gruppe der Formel (a) oder (b)
- R₂ und R₃ bedeuten Adamantyl oder R₂ und R₃ bilden gemeinsam mit dem angrenzenden aromatischen Ring einen gesättigten, 5- oder 6-gliedrigen Ring, der gegebenenfalls mit Methylgruppen substituiert und/oder gegebenenfalls durch ein Sauerstoffatom oder Schwefelatom unterbrochen ist.

15. Verbindungen nach einem der vorhergehenden Ansprüche zur Verwendung als Arzneimittel.

16. Verbindungen nach Anspruch 15 für die Verwendung als Arzneimittel, das zur Behandlung von dermatologischen Erkrankungen, die mit einer Verhornungsstörung verbunden sind, die auf der Differenzierung und Proliferation beruht, insbesondere zur Behandlung von Acne vulgaris, Acne comedonica, polymorpher Acne, Acne rosaceae, nodulocystischer Acne, Acne conglobata, Acne senilis und sekundären Akneformen, wie Acne solaris, Acne medicamentosa oder Acne professionalis; zur Behandlung weiterer Verhornungsstörungen, wie Ichtyosis, ichtyosisartigen Zuständen, der Darier Krankheit, Palmoplantarkeratosen, Leukoplakien und leucoplakieformen Zuständen und Lichen der Haut oder der Schleimhäute (buccal); zur Behandlung weiterer dermatologischer Erkrankungen mit entzündlicher Komponente und/oder immunoallergischer Komponente, die mit einer Verhornungsstörung verbunden sind, insbesondere alle Arten von Psoriasis der Haut, der Schleimhäute oder der Nägel, Psoriasis arthropathica, Atopie der Haut, wie Ekzemen, Atopie der Atemwege oder Hypertrophie des Zahnfleisches; wobei die Verbindungen auch bei einigen entzündlichen Störungen eingesetzt werden können, die nicht mit einer Verhornungsstörung verbunden sind; zur Behandlung aller Proliferationen der Dermis oder Epidermis, die gutartig oder bösartig und gegebenenfalls viralen Ursprungs sein können, wie Verrucae vulgares, Verrucae planae und Epidermodysplasia verruciformis, Papillomatosis oralis oder florida und Proliferationen, die von UV-Strahlung induziert sein können, insbesondere im Falle von Epithelioma basocellulare und spinocellulare; zur Behandlung von weiteren dermatologischen Störungen, wie bullösen Dermatosen und Kollagenerkrankungen; zur Behandlung einiger ophthalmologischer Störungen, insbesondere Corneopathien; zur Bekämpfung oder Behandlung der Hautalterung, die lichtinduziert oder altersbedingt sein kann, oder zur Verminderung von Pigmentierungen und aktinischen Keratosen, oder beliebiger Erkrankungen, die mit einer altersbedingten oder aktinischen Alterung zusammenhängen; Zur Vorbeugung oder Bekämpfung von Wundmalen der epidermalen und/ oder der dermalen Atrophie, die durch lokal oder systemisch verabreichte Corticosteroide hervorgerufen wird, oder beliebiger weiterer Formen der Atrophie der Haut, zur Vorbeugung oder Behandlung von Störungen der Wundheilung oder zur Vorbeugung oder Behandlung von Streifen; zur Förderung der Wundheilung, zur Bekämpfung von Funktionsstörungen der Talgdrüsen, wie Hyperseborrhoe bei Akne oder Seborrhoe simplex; zur Behandlung oder Vorbeugung von kanzerösen oder präkanzerösen Zuständen, insbesondere promyelozytärer Leukämie; zur Behandlung von entzündlichen Erkrankungen, wie Arthritis, zur Behandlung von Hautkrankheiten viralen Ursprungs oder allgemeinen Erkrankungen viralen Ursprungs; zur Vorbeugung oder Behandlung von Alopezie; zur Behandlung von dermatologischen Erkrankungen mit Immunkomponente; zur Behandlung von Erkrankungen des cardiovaskulären Systems, wie Arteriosklerose, Bluthochdruck, insulinunabhängiger Diabetes oder Adipositas oder zur Behandlung von Hautstörungen, die von einer Exposition gegenüber UV-Strahlung herrühren, vorgesehen ist.

17. Pharmazeutische Zusammensetzung, **dadurch gekennzeichnet, dass** sie in einem pharmazeutisch akzeptablen Träger mindestens eine Verbindung nach einem der Ansprüche 1 bis 14 enthält.

18. Zusammensetzung nach Anspruch 17, **dadurch gekennzeichnet, dass** die Konzentration der Verbindung(en) nach einem der Ansprüche 1 bis 14 im Bereich von 0,001 bis 5 Gew.-%, bezogen auf die gesamte Zusammensetzung, liegt.

19. Kosmetische Zusammensetzung, **dadurch gekennzeichnet, dass** sie in einem kosmetisch akzeptablen Träger mindestens eine Verbindung nach einem der Ansprüche 1 bis 14 enthält.

20. Zusammensetzung nach Anspruch 19, **dadurch gekennzeichnet, dass** die Konzentration der Verbindung(en) nach einem der Ansprüche 1 bis 14 im Bereich von 0,001 bis 3 Gew.-%, bezogen auf die gesamte Zusammensetzung, liegt.

21. Verwendung einer kosmetischen Zusammensetzung nach einem der Ansprüche 19 oder 20 zur Körperpflege oder Haarpflege.

## Claims

1. Compounds, **characterized in that** they correspond to the general formula (I) below: 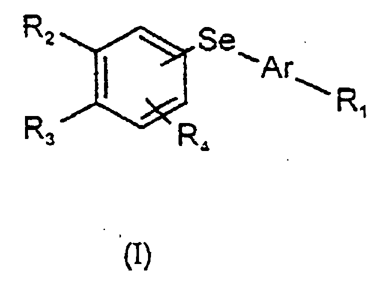 in which:
- R₁ represents:
(i)a -CH₃ radical,
(ii)a radical -CH₂-O-R₅,
(iii)a radical -COR₆,
R₅ and R₆ having the meanings given below,
- Ar represents a radical chosen from the radicals of formulae (a)-(e) below: 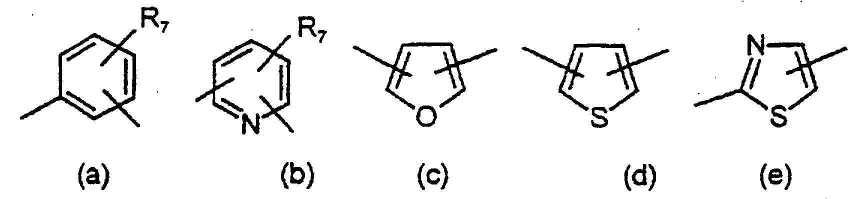 R₇ having the meaning given below,
- R₂ and R₃, which may be identical or different, independently represent a radical chosen from:
(i)a hydrogen atom,
(ii)a radical chosen from tert-butyl, 1-methylcyclohexyl and 1-adamantyl radicals,
(iii)a radical -OR₈, R₈ having the meaning given below,
(iv)a polyether radical containing 1 to 6 carbon atoms and 1 to 3 oxygen or sulphur atoms,
it being understood that at least one of the radicals R₂ or R₃ represents a radical (ii),
- R₂ and R₃ taken together can form, with the adjacent aromatic ring, a 5- or 6-membered saturated ring optionally substituted with methyl groups and/or optionally interrupted with an oxygen or sulphur atom,
- R₄ represents a hydrogen atom, a halogen atom, an alkyl radical containing 1 to 6 carbon atoms, a radical OR₉, a polyether radical containing 1 to 6 carbon atoms and 1 to 3 oxygen or sulphur atoms or a radical COR₁₀,
R₉ and R₁₀ having the meanings given below,
- R₅ represents a hydrogen atom, an alkyl radical containing 1 to 6 carbon atoms or a radical COR₁₁,
R₁₁ having the meaning given below,
- R₆ represents a radical chosen from:
(i)a hydrogen atom,
(ii)an alkyl radical containing 1 to 6 carbon atoms,
(iii)a radical OR₁₂,
R₁₂ having the meaning given below,
(iv)a radical of formula 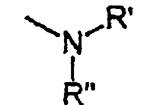 R' and R" having the meanings given below,
- R₇ represents a hydrogen atom, a halogen atom, an alkyl radical containing 1 to 6 carbon atoms, a nitro radical, a radical OR₁₃, a polyether radical containing 1 to 6 carbon atoms and 1 to 3 oxygen or sulphur atoms or a radical of the following formula: 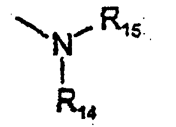 R₁₃ , R₁₄ and R₁₅ having the meanings given below,
- R₈ represents a hydrogen atom, an alkyl radical containing 1 to 6 carbon atoms, an optionally substituted aryl radical, an optionally substituted aralkyl radical, a monohydroxyalkyl radical containing 1 to 6 carbon atoms or a polyhydroxyalkyl radical containing 2 to 6 carbon atoms and 2 to 5 hydroxyl groups or an acyl radical containing 1 to 4 carbon atoms,
- R₉ represents a hydrogen atom, an alkyl radical containing 1 to 6 carbon atoms, an optionally substituted aryl radical, an optionally substituted aralkyl radical, a monohydroxyalkyl radical containing 1 to 6 carbon atoms or a polyhydroxyalkyl radical containing 2 to 6 carbon atoms and 2 to 5 hydroxyl groups, an acyl radical containing 1 to 4 carbon atoms, a radical - (CH₂)ₙ-COOR₁₆ or a radical -(CH₂)ₙ-X,
n, R₁₆ and X having the meanings given below,
- R₁₀ and R₁₁, which may be identical or different, represent an alkyl radical containing 1 to 6 carbon atoms,
- R₁₂ represents a hydrogen atom, an alkyl radical containing 1 to 6 carbon atoms, an optionally substituted aryl or aralkyl radical, a monohydroxyalkyl radical containing 1 to 6 carbon atoms or a polyhydroxyalkyl radical containing 2 to 6 carbon atoms and 2 to 5 hydroxyl groups,
- R' and R", which may be identical or different, represent a hydrogen atom, an alkyl radical containing 1 to 6 carbon atoms, an optionally substituted aryl radical or an amino acid residue,
or alternatively R' and R" taken together can form, with the nitrogen atom, a heterocycle,
- R₁₃ represents a hydrogen atom or an alkyl radical containing 1 to 6 carbon atoms,
- R₁₄ and R₁₅, which may be identical or different, represent a hydrogen atom or an alkyl radical containing 1 to 6 carbon atoms,
- R₁₆ represents a hydrogen atom or an alkyl radical containing 1 to 6 carbon atoms,
- n represents an integer between 1 and 12 inclusive,
- X represents a halogen atom,
and the optical and geometrical isomers of the said compounds of formula (I), as well as the salts thereof.

2. Compounds according to Claim 1, **characterized in that** they are in the form of salts of an alkali metal or alkaline-earth metal, of zinc, of an organic amine or of an inorganic or organic acid.

3. Compounds according to either of Claims 1 and 2, **characterized in that** the alkyl radicals containing 1 to 6 carbon atoms are chosen from methyl, ethyl, isopropyl, butyl and tert-butyl radicals.

4. Compounds according to one of the preceding claims, **characterized in that** the monohydroxyalkyl radicals containing 1 to 6 carbon atoms correspond to radicals containing 2 or 3 carbon atoms, in particular a 2-hydroxyethyl, 2-hydroxypropyl or 3-hydroxypropyl radical, it being possible for the monohydroxyalkyl radical to be protected in the form of acetyl or tertbutyldimethylsilyl.

5. Compounds according to one of the preceding claims, **characterized in that** the polyhydroxyalkyl radicals containing 2 to 6 carbon atoms and 2 to 5 hydroxyl groups are chosen from 2,3-dihydroxypropyl, 2,3,4-trihydroxybutyl and 2,3,4,5-tetrahydroxypentyl radicals or a pentaerythritol residue, it being possible for the hydroxyl groups to be protected in the form of acetyls or tert-butyldimethylsilyls.

6. Compounds according to one of the preceding claims, **characterized in that** the aryl radicals correspond to a phenyl radical, optionally substituted with at least one halogen, one hydroxyl or one nitro function.

7. Compounds according to one of the preceding claims, **characterized in that** the aralkyl radicals are chosen from benzyl and phenethyl radicals optionally substituted with at least one halogen, one hydroxyl or one nitro function.

8. Compounds according to one of the preceding claims, **characterized in that** the acyl radicals containing 1 to 4 carbon atoms are chosen from an acetyl radical or a propionyl radical.

9. Compounds according to any one of the preceding claims, **characterized in that** the polyether radicals containing 1 to 6 carbon atoms and 1 to 3 oxygen or sulphur atoms are chosen from methoxymethyl ether, methoxyethoxymethyl ether and methylthiomethyl ether radicals.

10. Compounds according to any one of the preceding claims, **characterized in that** the amino acid residues are chosen from the group consisting of residues derived from lysine, glycine or from aspartic acid.

11. Compounds according to any one of the preceding claims, **characterized in that** the heterocyclic radicals are chosen from the group consisting of piperidino, morpholino, pyrrolidino and piperazino radicals, optionally substituted in position 4 with a C₁-C₆ alkyl radical or with a mono- or polyhydroxyalkyl radical.

12. Compounds according to Claim 1, **characterized in that** they are taken, alone or as mixtures, from the group consisting of:
ethyl 4-(3,5,5,8,8-pentamethyl-5,6,7,8-tetrahydro-2-naphthylselanyl)benzoate,
4-(3,5,5,8,8-pentamethyl-5,6,7,8-tetrahydro-2-naphthylselanyl)benzoic acid,
ethyl 6-(3,5,5,8,8-pentamethyl-5,6,7,8-tetrahydro-2-naphthylselanyl)nicotinate,
6-(3,5,5,8,8-pentamethyl-5,6,7,8-tetrahydro-2-naphthylselanyl)nicotinic acid,
ethyl 6-(5,5,8,8-tetramethyl-3-propoxy-5,6,7,8-tetrahydro-2-naphthylselanyl)nicotinate,
6-(5,5,8,8-tetramethyl-3-propoxy-5,6,7,8-tetrahydro-2-naphthylselanyl)nicotinic acid,
3-(4-tert-butylphenylselanyl)benzoic acid,
6-(4-tert-butylphenylselanyl)nicotinic acid,
4-(4-tert-butylphenylselanyl)benzoic acid,
4-(4,4-dimethylthiochroman-8-ylselanyl)benzoic acid,
3-(4,4-dimethylthiochroman-8-ylselanyl)benzoic acid,
6-(4,4-dimethylthiochroman-8-ylselanyl)nicotinic acid,
4-(5,5,8,8-tetramethyl-5,6,7,8-tetrahydro-2-naphthylselanyl)benzoic acid,
3-(5,5,8,8-tetramethyl-5,6,7,8-tetrahydro-2-naphthylselanyl)benzoic acid,
6-(5,5,8,8-tetramethyl-5,6,7,8-tetrahydro-2-naphthylselanyl)nicotinic acid,
4-[5-adamantan-1-yl-4-(2-methoxyethoxymethoxy)-2-methylphenylselanyl]benzoic acid,
3-[5-adamantan-1-yl-4-(2-methoxyethoxymethoxy)-2-methylphenylselanyl]benzoic acid,
6-(4-methoxyethoxymethoxy-5,5,8,8-tetramethyl-5,6,7,8-tetrahydro-2-naphthylselanyl)nicotinic acid,
3-(4-methoxyethoxymethoxy-5,5,8,8-tetramethyl-5,6,7,8-tetrahydro-2-naphthylselanyl)benzoic acid,
4-(4-methoxyethoxymethoxy-5,5,8,8-tetramethyl-5,6,7,8-tetrahydro-2-naphthylselanyl)-3-methoxybenzoic acid,
3-(4-methoxyethoxymethoxy-5,5,8,8-tetramethyl-5,6,7,8-tetrahydro-2-naphthylselanyl)-4-methoxybenzoic acid,
6-(4-methoxymethoxy-5,5,8,8-tetramethyl-5,6,7,8-tetrahydro-2-naphthylselanyl)nicotinic acid,
6-(3-methoxyethoxymethoxy-5,5,8,8-tetramethyl-5,6,7,8-tetrahydro-2-naphthylselanyl)nicotinic acid,
2-(3-methoxyethoxymethoxy-5,5,8,8-tetramethyl-5,6,7,8-tetrahydro-2-naphthylselanyl)nicotinic acid,
4-(3-methoxyethoxymethoxy-5,5,8,8-tetramethyl-5,6,7,8-tetrahydro-2-naphthylselanyl)benzoic acid,
3-(3-methoxyethoxymethoxy-5,5,8,8-tetramethyl-5,6,7,8-tetrahydro-2-naphthylselanyl)benzoic acid,
6-(3,5-di-tert-butyl-2-methoxymethoxyphenylselanyl)-nicotinic acid,.
2-(3,5-di-tert-butyl-2-methoxymethoxyphenylselanyl)-nicotinic acid,
4-(3,5-di-tert-butyl-2-methoxymethoxyphenylselanyl)-benzoic acid,
3-(3,5-di-tert-butyl-2-methoxymethoxyphenylselanyl)-benzoic acid,
6-[4-adamantan-1-yl-3-benzyloxyphenylselanyl]nicotinic acid,
6-(3,5-di-tert-butyl-2-benzyloxyphenylselanyl)nicotinic acid,
3-methoxy-4-(4-benzyloxy-5,6,7,8-tetrahydro-5,5,8,8-tetramethyl-2-naphthylselanyl)benzoic acid,
4-(4-benzyloxy-5,6,7,8-tetrahydro-5,5,8,8-tetramethyl-2-naphthylselanyl)benzoic acid,
6-(4-benzyloxy-5,6,7,8-tetrahydro-5,5,8,8-tetramethyl-2-naphthylselanyl)nicotinic acid,
3-methoxy-4-(3-benzyloxy-5,6,7,8-tetrahydro-5,5,8,8-tetramethyl-2-naphthylselanyl)benzoic acid,
6-(3-benzyloxy-5,6,7,8-tetrahydro-5,5,8,8-tetramethyl-2-naphthylselanyl)nicotinic acid,
4-(3-hexyloxy-5,6,7,8-tetrahydro-5,5,8,8-tetramethyl-2-naphthylselanyl)-3-methoxybenzoic acid,
6-(3-hexyloxy-5,6,7,8-tetrahydro-5,5,8,8-tetramethyl-2-naphthylselanyl)nicotinic acid,
4-(5-adamantan-1-yl-4-benzyloxy-2-methylphenylselanyl)-benzoic acid,
6-[3-(5-hydroxypentyloxy)-5,5,8,8-tetramethyl-5,6,7,8-tetrahydro-2-naphthylselanyl]nicotinic acid,
ethyl 4-(5,5,8,8-tetramethyl-5,6,7,8-tetrahydro-2-naphthylselanyl)benzoate,
ethyl 4-(3-methoxyethoxymethoxy-5,5,8,8-tetramethyl-5,6,7,8-tetrahydro-2-naphthylselanyl)benzoate,
ethyl 4-(3-hydroxy-5,5,8,8-tetramethyl-5,6,7,8-tetrahydro-2-naphthylselanyl)benzoate,
4-(3-hydroxy-5,5,8,8-tetramethyl-5,6,7,8-tetrahydro-2-naphthylselanyl)benzoic acid,
ethyl 6-(3-methoxyethoxymethoxy-5,5,8,8-tetramethyl-5,6,7,8-tetrahydro-2-naphthylselanyl)nicotinate,
ethyl 6-(3-hydroxy-5,5,8,8-tetramethyl-5,6,7,8-tetrahydro-2-naphthylselanyl)nicotinate,
6-(3-hydroxy-5,5,8,8-tetramethyl-5,6,7,8-tetrahydro-2-naphthylselanyl)nicotinic acid,
ethyl 6-[3-(3-ethoxycarbonylpropoxy)-5,5,8,8-tetramethyl-5,6,7,8-tetrahydro-2-naphthylselanyl]nicotinate,
6-[3-(3-carboxypropoxy)-5,5,8,8-tetramethyl-5,6,7,8-tetrahydro-2-naphthylselanyl]nicotinic acid,
ethyl 4-[3-(3-ethoxycarbonylpropoxy)-5,5,8,8-tetramethyl-5,6,7,8-tetrahydro-2-naphthylselanyl]benzoate,
4-[3-(3-carboxypropoxy)-5,5,8,8-tetramethyl-5,6,7,8-tetrahydro-2-naphthylselanyl]benzoic acid,
ethyl 4-[3-(7-methoxycarbonylheptyloxy)-5,5,8,8-tetramethyl-5,6,7,8-tetrahydro-2-naphthylselanyl]benzoate,
4-[3-(7-carboxyheptyloxy)-5,5,8,8-tetramethyl-5,6,7,8-tetrahydro-2-naphthylselanyl]benzoic acid,
ethyl 6-[3-(7-methoxycarbonylheptyloxy)-5,5,8,8-tetramethyl-5,6,7,8-tetrahydro-2-naphthylselanyl]nicotinate,
6-[3-(7-carboxyheptyloxy)-5,5,8,8-tetramethyl-5,6,7,8-tetrahydro-2-naphthylselanyl]nicotinic acid,
ethyl 6-[3-(2-acetoxyethoxy)-5,5,8,8-tetramethyl-5,6,7,8-tetrahydro-2-naphthylselanyl]nicotinate,
6-[3-(2-hydroxyethoxy)-5,5,8,8-tetramethyl-5,6,7,8-tetrahydro-2-naphthylselanyl]nicotinic acid,
ethyl 4-[3-(2-acetoxyethoxy)-5,5,8,8-tetramethyl-5,6,7,8-tetrahydro-2-naphthylselanyl]benzoate,
4-[3-(2-hydroxyethoxy)-5,5,8,8-tetramethyl-5,6,7,8-tetrahydro-2-naphthylselanyl]benzoic acid,
6-(3-adamantan-1-yl-4-methoxyphenylselanyl)nicotinic acid,
[6-(3,5,5,8,8-pentamethyl-5,6,7,8-tetrahydro-2-naphthylselanyl)-3-pyridyl]methanol,
N-ethyl-6-(3,5,5,8,8-pentamethyl-5,6,7,8-tetrahydro-2-naphthylselanyl)nicotinamide,
morpholin-4-yl-[6-(3,5,5,8,8-pentamethyl-5,6,7,8-tetrahydro-2-naphthylselanyl)-3-pyridyl]methanone,
N-(4-hydroxyphenyl)-6-(3,5,5,8,8-pentamethyl-5,6,7,8-tetrahydro-2-naphthylselanyl)nicotinamide.
6-(3,5,5,8,8-pentamethyl-5,6,7,8-tetrahydro-2-naphthylselanyl)pyridine-3-carbaldehyde.

13. Compounds according to Claim 1, **characterized in that** they have at least one of the following characteristics:
- R₁ represents a radical COR₆
- Ar represents a radical of formula (a) or (b)
- R₂ or R₃ represents an adamantyl radical or R₂ and R₃ taken together form, with the adjacent aromatic ring, a 5- or 6-membered saturated ring optionally substituted with methyl groups and/or optionally interrupted with an oxygen or sulphur atom.

14. Compounds according to Claim 13, **characterized in that** they have all of the following characteristics:
- R₁ represents a radical COR₆
- Ar represents a radical of formula (a) or (b)
- R₂ or R₃ represents an adamantyl radical or R₂ and R₃ taken together form, with the adjacent aromatic ring, a 5- or 6-membered saturated ring optionally substituted with methyl groups and/or optionally interrupted with an oxygen or sulphur atom.

15. Compounds according to any one of the preceding claims, for use as medicinal products.

16. Compounds according to Claim 15, for use as medicinal products intended for treating dermatological complaints associated with a keratinization disorder which has a bearing on differentiation and on proliferation, in particular for treating common acne, comedones, polymorphonuclear leukocytes, rosacea, nodulocystic acne, acne conglobata, senile acne, secondary acnes such as solar, medication-related or occupational acne; for treating other types of keratinization disorder, in particular ichthyosis, ichthyosiform states, Darier's disease, palmoplantar keratoderma, leucoplasias and leucoplasiform states, and cutaneous or mucous (buccal) lichen; for treating other dermatological complaints associated with a keratinization disorder with an inflammatory and/or immunoallergic component and, in particular, all forms of psoriasis, whether it is cutaneous, mucous or ungual psoriasis and even psoriatic rheumatism, or alternatively cutaneous atopy, such as eczema or respiratory atopy or alternatively gingival hypertrophy; the compounds can also be used in certain inflammatory complaints which have no keratinization disorder; for treating all dermal or epidermal proliferations, whether benign or malignant and whether they are of viral origin or otherwise, such as common warts, flat warts and verruciform epidermodysplasia, oral or florid papillomatoses and proliferations which may be induced by ultraviolet radiation, in particular in the case of basocellular and spinocellular epithelioma; for treating other dermatological disorders such as bullosis and collagen diseases; for treating certain ophthalmological disorders, in particular corneopathies; for repairing or combating ageing of the skin, whether this is light-induced or chronological ageing, or for reducing actinic keratoses and pigmentations, or any pathologies associated with chronological or actinic ageing; for preventing or curing the stigmata of epidermal and/or dermal atrophy induced by local or systemic corticosteroids, or any other form of cutaneous atrophy; for preventing or treating cicatrization disorders or for preventing or repairing stretchmarks, or alternatively for promoting cicatrization; for combating disorders of sebaceous functioning such as the hyperseborrhoea of acne or simple seborrhoea; in the treatment or prevention of cancerous or precancerous states, more particularly promyelocyte leukaemias; in the treatment of inflammatory complaints such as arthritis; in the treatment of any general or skin complaint of viral origin; in the prevention or treatment of alopecia; in the treatment of dermatological complaints having an immunological component; in the treatment of complaints of the cardiovascular system such as arteriosclerosis, hypertension, non-insulin-dependent diabetes and obesity; in the treatment of skin disorders due to an exposure to U.V. radiation.

17. Pharmaceutical composition, **characterized in that** it comprises, in a pharmaceutically acceptable support, at least one of the compounds as defined in any one of Claims 1 to 14.

18. Composition according to Claim 17, **characterized in that** the concentration of compound(s) according to one of Claims 1 to 14 is between 0.001% and 5% by weight relative to the composition as a whole.

19. Cosmetic composition, **characterized in that** it comprises, in a cosmetically acceptable support, at least one of the compounds as defined in any one of Claims 1 to 14.

20. Composition according to Claim 19, **characterized in that** the concentration of compound(s) according to one of Claims 1 to 14 is between 0.001% and 3% by weight relative to the composition as a whole.

21. Use of a cosmetic composition as defined in either of Claims 19 and 20, for body or hair hygiene.
